# EUROPEAN PATENT SPECIFICATION

(11) **EP 4 330 914 B1**
(45) Date of publication and mention of the grant of the patent: **08.07.2026**
(21) Application number: 22875015.4
(22) Date of filing: 28.09.2022
(51) Int. Cl.: G06T 7/00

(54) **METHODS AND SYSTEMS FOR IMAGE PROCESSING**
VERFAHREN UND SYSTEME ZUR BILDVERARBEITUNG
PROCÉDÉS ET SYSTÈMES DE TRAITEMENT D'IMAGE

(30) Priority: 28.09.2021 CN 202111144672; 28.10.2021 CN 202111261975; 31.12.2021 CN 202111678066
(43) Date of publication of application: 06.03.2024
(73) Proprietor: Shanghai United Imaging Healthcare Co., Ltd., Shanghai 201807 (CN)
(72) Inventor: YE, Qing, Shanghai 201807 (CN); ZHAO, Yizhang, Shanghai 201807 (CN); LIU, Hui, Shanghai 201807 (CN); MA, Runxia, Shanghai 201807 (CN); JI, Zijun, Shanghai 201807 (CN); QU, Yinghan, Shanghai 201807 (CN); LI, Jinhua, Shanghai 201807 (CN)
(74) Representative: Wang, Bo
(86) International application number: PCT/CN2022/122230
(87) International publication number: WO 2023/051632

(56) References cited:
- CN-A- 108 932 741
- CN-A- 113 822 298
- CN-A- 113 989 231
- CN-A- 114 283 927
- JP-A- 2020 076 584
- US-A1- 2020 320 753
- US-A1- 2021 118 202
- MABROUK R ET AL: "Extraction of time activity curves from gated FDG-PET images for small animals heart studies", COMPUTERIZED MEDICAL IMAGING AND GRAPHICS, PERGAMON PRESS, NEW YORK, NY, US, vol. 36, no. 6, 10 May 2012 (2012-05-10), pages 484 - 491, XP028425517, ISSN: 0895-6111, [retrieved on 20120517], DOI: 10.1016/J.COMPMEDIMAG.2012.05.002
- LIU GUOBING ET AL: "Ultra-low-activity total-body dynamic PET imaging allows equal performance to full-activity PET imaging for investigating kinetic metrics of 18F-FDG in healthy volunteers", EUROPEAN JOURNAL OF NUCLEAR MEDICINE AND MOLECULAR IMAGING, SPRINGER BERLIN HEIDELBERG, BERLIN/HEIDELBERG, vol. 48, no. 8, 22 January 2021 (2021-01-22), pages 2373 - 2383, XP037494092, ISSN: 1619-7070, [retrieved on 20210122], DOI: 10.1007/S00259-020-05173-3
- CHERRY SIMON R. ET AL: "Total-body imaging: Transforming the role of positron emission tomography", SCIENCE TRANSLATIONAL MEDICINE(SUPPLEMENTARY MATERIALS), vol. 9, no. 381, 15 March 2017 (2017-03-15), XP093290399, ISSN: 1946-6234, DOI: 10.1126/scitranslmed.aaf6169

## Description

### TECHNICAL FIELD

The present disclosure relates to the field of imaging, in particular to methods and systems for image processing.

### BACKGROUND

Positron emission computed tomography (PET) imaging technology can directly or indirectly reflect the biological metabolic process by injecting a tracer into a living organism, so as to achieve the purpose of diagnosis and analysis, and provide an effective basis for the early diagnosis and prevention of diseases. Compared with an image reconstruction analysis only for PET in a static time window, dynamic PET can be used to perform a quantitative analysis on a real metabolic level of tissues or organs of an organism. Alternative protocols involving a dynamic obtaining of images can measure tracer kinetics more completely. A kinetic model can describe an exchange process of a tracer between different compartments during scanning by kinetic-related parameters (e.g., an input function (IF), a time-activity curve (TAC), etc.), which has important clinical significances.

The time-activity curve can reflect a concentration of a radioactive tracer in a specific region (such as a tissue or plasma). A Y-axis represents the concentration and an X-axis represents time. In some embodiments, the time-activity curve may be used as an input function of the plasma in the kinetic model to facilitate further analysis of drug metabolisms, such as solving kinetic parameters, or the like.

In traditional technology, it is generally to collect PET-CT images to obtain computed tomography (CT) image sequences, draw on the CT images to obtain regions of interest, map the drawn regions of interest to the PET images, analyze the mapped regions of interest in the PET images, obtain kinetic parameters of the mapped regions of interest, and provide them to doctors as a diagnostic basis.

Thus, it is desirable to provide methods and systems for image processing to improve the accuracy of the time-activity curve, ensure the convenience of obtaining the input function and better match actual needs, and efficiently obtain the kinetic parameters.
JP 2020 076584 A relates to a medical image processing apparatus.
MABROUK R ET AL: "Extraction of time activity curves from gated FOG-PET images for small animals heart studies", COMPUTERIZED MEDICAL IMAGING AND GRAPHICS, PERGAMON PRESS, NEW YORK, NY, US, vol. 36, no. 6, 10 May 2012 (2012-05-10), pages 484-491, XP028425517, ISSN: 0895-6111, DOI: 10. 10 1 6/J. COMPMEDIMAG.2012.05.002, relates to a new approach to extract the input function and the tissue time activity curve from dynamic ECG-gated 18F-FDG PET images.
LIU GUOBING ET AL: "Ultra-low-activity total-body dynamic PET imaging allows equal performance to full-activity PET imaging for investigating kinetic metrics of 18F-FDG in healthy volunteers", EUROPEAN JOURNAL OF NUCLEAR MEDICINE AND MOLECULAR IMAGING, SPRINGER BERLIN HEIDELBERG, BERLIN/HEIDELBERG, vol. 48, no. 8, 22 January 2021 (2021-01-22), pages 2373-2383, XP037494092, ISSN: 1619-7070, DOI: 10.1007/S00259-020-05173-3, relates to a total-body dynamic PET imaging using 10x reduction of injected activity.

### SUMMARY

The invention is set out in the appended set of claims.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating an exemplary medical system according to some embodiments of the present disclosure;
FIG. 2 is a schematic diagram illustrating an exemplary computing device on which at least a portion of the medical system may be implemented according to some embodiments of the present disclosure;
FIG. 3 is a flowchart illustrating an exemplary process for determining a time-activity curve according to some embodiments of the present disclosure;
FIG. 4 is a flowchart illustrating an exemplary process for training an extraction model according to some embodiments of the present disclosure;
FIG. 5 is a schematic diagram illustrating an exemplary system for determining the time-activity curve according to some embodiments of the present disclosure;
FIG. 6 is a flowchart illustrating an exemplary process for determining an input function according to some embodiments of the present disclosure;
FIG. 7 is a flowchart illustrating an exemplary process for determining a kinetic parametric image according to some embodiments of the present disclosure;
FIG. 8 is a flowchart illustrating an exemplary process for determining one or more values of one or more kinetic parameters according to some embodiments of the present disclosure;
FIG. 9 is a flowchart illustrating an exemplary process for training a segmentation model according to other embodiments of the present disclosure;
FIG. 10 is a schematic diagram illustrating an exemplary system for determining kinetic parameters according to some embodiments of the present disclosure;
FIG. 11 is a flowchart illustrating an exemplary process for determining an input function according to some embodiments of the present disclosure;
FIG. 12 is a schematic diagram illustrating an exemplary time-activity curve to be eliminated according to some embodiments of the present disclosure;
FIG. 13 is a schematic diagram illustrating a third interface for determining an input function according to some embodiments of the present disclosure;
FIG. 14 is a schematic diagram of a second interface for determining an input function according to some embodiments of the present disclosure;
FIG. 15 is a schematic diagram of a fourth interface for determining an input function according to some embodiments of the present disclosure;
FIG. 16 is a schematic diagram illustrating an exemplary device for determining dynamic parameters according to some embodiments of the present disclosure; and
FIG. 17 is a schematic diagram illustrating an exemplary device for determining dynamic parameters according to other embodiments of the present disclosure.

### DETAILED DESCRIPTION

In order to more clearly explain the technical scheme of the embodiment of the present disclosure, the drawings required in the description of the embodiment are briefly introduced below. Obviously, the drawings in the following description are only some examples or embodiments of the present disclosure. For those skilled in the art, the present disclosure can also be applied to other similar situations according to these drawings without paying creative labor. Unless obviously obtained from the context or the context illustrates otherwise, the same numeral in the drawings refers to the same structure or operation.

It should be understood that the "system", "device", "unit" and/or "module" used herein is a method for distinguishing different components, elements, components, parts, or assemblies at different levels. However, if other words may achieve the same purpose, the words may be replaced by other expressions.

As shown in the present disclosure and claims, unless the context clearly prompts the exception, "a", "one", and/or "the" is not specifically singular, and the plural may be included. In general, the terms "comprises" "comprising" "includes" and/or "including" only indicate that the steps and units that have been clearly identified are included. the steps and units do not constitute an exclusive list, and the method or device may also include other steps or units.

The flowcharts are used in the present disclosure to illustrate operations performed by the system according to the embodiment of the present disclosure. It should be understood that the foregoing or following operations may not be necessarily performed exactly in order. Instead, each operation may be processed in reverse or simultaneously. Moreover, other operations may also be added into these procedures, or one or more steps may be removed from these procedures.

A reference standard method for determining a time-activity curve may obtain concentration information by simultaneously performing blood collection and imaging. However, since blood collection is an invasive detection method, in some embodiments, a non-invasive method may be considered to obtain the time-activity curve.

In some embodiments, a time-activity curve of a blood pool part may be determined by an image-derived input function (IDIF) method as an input function of the plasma. Specifically, it is necessary to draw a region of interest (ROI) in an image. It is difficult to draw the region of interest, and accuracy of the drawing is directly related to accuracy of the time-activity curve. In addition, due to different image positions, it is usually necessary to perform a correction of a partial volume effect, which may increase difficulty of determining the time-activity curve.

In view of this, in some embodiments of the present disclosure, a method for determining a time-activity curve based on a machine learning model is provided, and the determined time-activity curve has high accuracy.

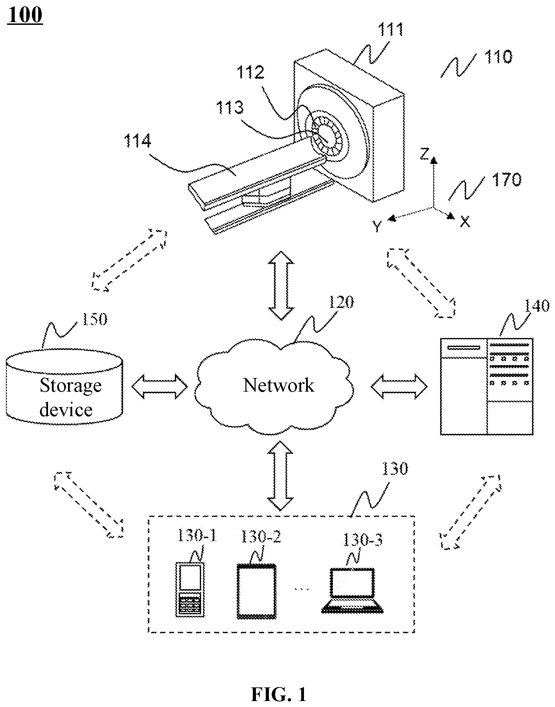

FIG. 1 is a schematic diagram illustrating an exemplary medical system according to some embodiments of the present disclosure.

A medical system 100 may include a medical device 110, a network 120, one or more terminals 130, a processing device 140, and a storage device 150. Components in the medical system 100 may be connected in various ways. As an example, the medical device 110 may be connected to the processing device 140 directly (as shown by a dashed two-way arrow connecting the medical device 110 and the processing device 140) or through the network 120. As another example, the storage device 150 may be connected to the medical device 110 directly (as shown by a dashed two-way arrow connecting the storage device 150 and the medical device 110) or through the network 120. As yet another example, the terminal 130 may be connected to the processing device 140 directly (as shown by a dashed two-way arrow connecting the terminal 130 and the processing device 140) or through the network 120. As yet another example, the terminal 130 may be connected to the storage device 150 directly (as shown by a dashed two-way arrow connecting the terminal 130 and the storage device 150) or through the network 120.

The medical device 110 may scan and/or treat an object. In some embodiments, the object may include a biological object and/or a non-biological object. For example, the object may include a specific part of the human body, such as the head, chest, abdomen, etc., or a combination thereof. As another example, the object may be a patient to be scanned by the medical device 110.

In some embodiments, the medical device 110 may scan the object to obtain data related to the object. For example, the medical device 110 may include a single modality scanning device and/or a multi-modality scanning device. The single modality scanning device may include, for example, an emission computed tomography (ECT) device, a positron emission tomography (PET) device, a single photon emission computed tomography (SPECT) device, an ultrasonic device, an X-ray device, a computed tomography (CT) device, a magnetic resonance imaging (MRI) device, or the like, or any combination thereof. The multi-modality scanning device may include, for example, an X-ray imaging-magnetic resonance imaging (X-ray-MRI) scanning device, a positron emission tomography-X-ray imaging (PET-X-ray) scanning device, a single photon emission computed tomography-magnetic resonance imaging (SPECT-MRI) scanning device, a positron emission tomography-computed tomography (PET-CT) scanning device, a positron emission tomography-magnetic resonance imaging (PET-MRI) scanning device, a digital subtraction angiography-magnetic resonance imaging (DSA-MRI) scanning device, or the like. The scanning devices provided above are for illustrative purposes only and are not intended to limit the scope of the present disclosure.

In some embodiments, the medical device 110 may be a PET device, including a gantry 111, a detector 112, a scanning region 113, and a scanning bed 114. The gantry 111 may be used to support the detector 112. The object may be placed on the scanning bed 114 and scanned by moving the scanning bed 114 into the scanning region 113. In some embodiments, the detector 112 may include one or more detector units. The detector 112 may include a scintillation detector (e.g., a cesium iodide detector), a gas detector, or the like. The detector 112 may be and/or include a single row detector, wherein a plurality of detector units may be disposed in a single row and/or a multi-row detector, wherein the plurality of detector units may be disposed in a plurality of rows.

The network 120 may include any suitable network that may facilitate an exchange of information and/or data of the medical system 100. In some embodiments, one or more components of the medical system 100 (e.g., the medical device 110, the terminal 130, the processing device 140, the storage device 150) may communicate information and/or data with one or more other components of the medical system 100 through network 120. For example, the processing device 140 may obtain scan data from the medical device 110 through the network 120. As another example, the processing device 140 may obtain a user instruction from the terminal 130 through the network 120. The network 120 may be and/or include a public network (e.g., the Internet), a private network (e.g., a local area network (LAN), a wide area network (WAN)), a wired network (e.g., a wireless local area network), an Ethernet, a wireless network (e.g., an 802.11 network, a Wi-Fi network), a cellular network (e.g., a long term evolution (LTE) network), a frame relay network, a virtual private network (VPN), a satellite network, a telephone network, a router, a hub, a server computer, or the like, or any combination thereof. As an example, the network 120 may include a cable network, a wired network, an optical fiber network, a telecommunications network, an intranet, a wireless local area network (WLAN), a metropolitan area network (MAN), a public switched telephone network (PSTN), a Bluetooth^{™} network, a ZigBee^{™} network, a near field communication (NFC) network, or the like, or any combination thereof. In some embodiments, network 120 may include one or more network access points. For example, the network 120 may include wired and/or wireless network access points, such as base stations and/or Internet switching points, through the access points one or more components of the medical system 100 may connect to the network 120 to exchange data and/or information.

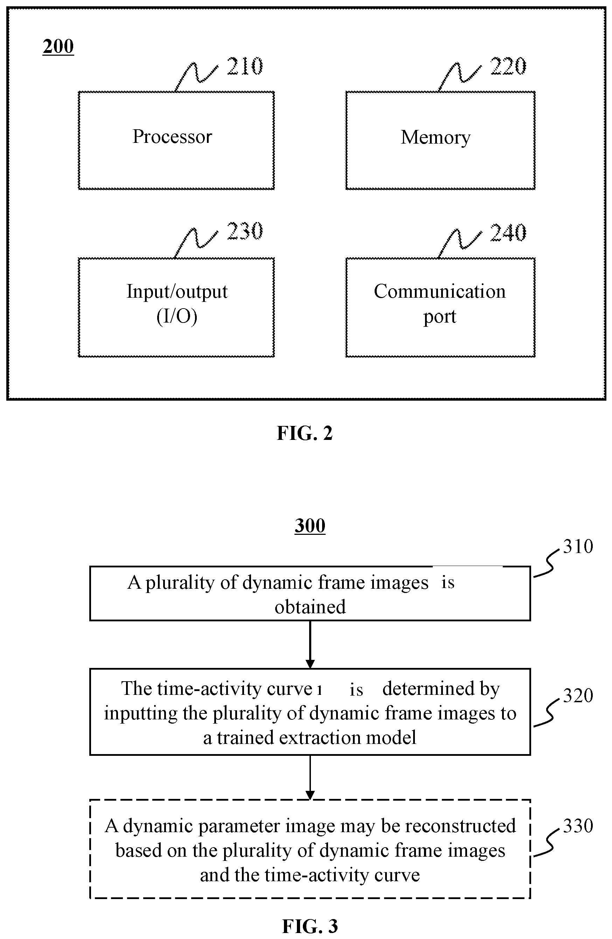

The terminal 130 may include a mobile device 131, a tablet computer 132, a laptop computer 133, or the like, or any combination thereof. In some embodiments, the mobile device 131 may include a smart home device, a wearable device, an intelligent mobile device, a virtual reality device, an augmented reality device, or the like, or any combination thereof. As an example, the terminal 130 may include a mobile device as shown in FIG. 3. In some embodiments, the smart home device may include a smart lighting device, a smart electrical control device, a smart monitoring device, a smart TV, a smart camera, a walkie-talkie, or the like, or any combination thereof. In some embodiments, the wearable device may include a bracelet, a pair of shoes, a pair of glasses, a helmet, a watch, an article of clothes, a backpack, a smart accessory, or the like, or any combination thereof. In some embodiments, the mobile device may include a mobile phone, a personal digital assistant (PDA), a game device, a navigation device, a point of sale (POS) device, a laptop computer, a tablet computer, a desktop computer, or the like, or any combination thereof. In some embodiments, the virtual reality device and/or the augmented reality device may include a virtual reality helmet, a pair of virtual reality glasses, a pair of virtual reality eye masks, an augmented reality helmet, a pair of augmented reality glasses, a pair of augmented reality eye masks, or the like, or any combination thereof. For example, the virtual reality device and/or the augmented reality device may include Google Glass^{™}, Oculus Rift^{™}, Hololens^{™}, Gear VR^{™}, or the like. In some embodiments, the one or more terminals 130 may be part of the processing device 140.

The processing device 140 may process data and/or information obtained from the medical device 110, the terminal 130 and/or the storage device 150. For example, the processing device 140 may obtain one or more sets of scan data of an object. As another example, the processing device 140 may obtain information of a series of frames by performing a framing processing on a plurality of sets of the scan data. As yet another example, the processing device 140 may determine metabolic information of the tracer in the object based on the information of a series of frames. As yet another example, the processing device 140 may replace one or more kinetic parameters in a kinetic model with one or more bounded functions according to physiological significance of the one or more kinetic parameters. As yet another example, the processing device 140 may determine one or more values of the one or more kinetic parameters by solving one or more values of the one or more bounded functions in the kinetic model based on the scan data. In some embodiments, the processing device 140 may be a single server or a server group. The server group may be centralized or distributed. In some embodiments, the processing device 140 may be a local component or a remote component relative to one or more other components of the medical system 100. For example, the processing device 140 may access information and/or data stored in the medical device 110, the terminal 130, and/or the storage device 150 through the network 120. As another example, the processing device 140 may be directly connected to the medical device 110, the terminal 130, and/or the storage device 150 to access stored information and/or data. In some embodiments, the processing device 140 may be implemented on a cloud platform. Merely by way of example, the cloud platform may include a private cloud, a public cloud, a hybrid cloud, a community cloud, a distributed cloud, an internal cloud, a multi-layer cloud, or the like, or any combination thereof. In some embodiments, the processing device 140 may be implemented by a computing device 200 with one or more components as shown in FIG. 2.

When applied on the medical system, the processing device 140 may obtain image data from the medical device through a transmission device. For example, the processing device 140 may obtain a user instruction through a transmission device. For another example, the processing device 140 may send a treatment queue, patient information, or the like, to an upper computer through the transmission device for display to a user. For another example, the processing device 140 may include a first processor and a second processor. The first processor may send data such as the treatment queue and the patient information to the upper computer through the Ethernet in TCP/IP protocol, and the second processor may send information such as mechanical parameters and hardware status of components of the medical device to the upper computer through the Ethernet in TCP/IP protocol. Applications running on the upper computer may present the data to the user.

The storage device 150 may store data, instructions and/or any other information. In some embodiments, the storage device 150 may store data obtained from the terminal 130 and/or the processing device 140. In some embodiments, the storage device 150 may store data and/or instructions that the processing device 140 may execute or use to perform an exemplary method described in the present disclosure. In some embodiments, the storage device 150 may include a mass memory, a removable memory, a volatile read-write memory, a read-only memory (ROM), or the like, or any combination thereof. The exemplary mass storage may include a magnetic disk, an optical disk, a solid state drive, or the like. The exemplary removable memory may include a flash drive, a floppy disk, an optical disk, a memory card, a compressed disk, a magnetic tape, or the like. The exemplary volatile read-write memory may include a random access memory (RAM). The exemplary RAM may include a dynamic random access memory (DRAM), a double data rate synchronous dynamic random access memory (DDR SDRAM), a static random access memory (SRAM), a thyristor random access memory (T-RAM), a zero capacitance random access memory (Z-RAM), or the like. The exemplary ROM may include a mask ROM (MROM), a programmable ROM (PROM), an erasable programmable ROM (EPROM), an electrically erasable programmable ROM (EEPROM), a compact disc ROM (CD-ROM), a digital versatile disc ROM, or the like. In some embodiments, the storage device 150 may be implemented on a cloud platform. As an example, the cloud platform may include a private cloud, a public cloud, a hybrid cloud, a community cloud, a distributed cloud, an internal cloud, a multi-layer cloud, or the like, or any combination thereof.

In some embodiments, the storage device 150 may be connected to the network 120 to communicate with the one or more other components of the medical system 100 (e.g., the processing device 140, the terminal 130). The one or more components of the medical system 100 may access data or instructions stored in the storage device 150 through the network 120. In some embodiments, the storage device 150 may be directly connected to or in communication with the one or more other components of the medical system 100 (e.g., the processing device 140, the terminal 130). In some embodiments, the storage device 150 may be part of the processing device 140.

In some embodiments, the medical device 110 may be provided with a coordinate system 170 to define a position (e.g., an absolute position, a position relative to another component) and/or a motion of a component of the medical device 110. For example, the coordinate system 170 may include an X-axis, a Y-axis, and a Z-axis. The X-axis and the Y-axis are axes in a horizontal direction, and the Z-axis is an axis in a vertical direction. As shown in FIG. 1, a positive direction of the X-axis may be a direction from a left side of the treatment bed to a right side when viewed from a front side of the medical device 110. A positive direction of the Y-axis may be a direction in which the treatment bed moves from inside of the medical device 110 to outside. A positive direction of the Z-axis may be a direction from a bottom of the medical device 110 (or a ground where the medical device 110 is located) to a top of the medical device 110. The coordinate system 170 is provided for illustrative purposes only. For example, the coordinate system 170 may also include other coordinate axes. For another example, directions of the X-axis, the Y-axis, and the Z-axis may be other directions, and the present disclosure is not limited.

It should be noted that the above description is provided for illustrative purposes only and is not intended to limit the scope of the present disclosure. For those skilled in the art, various modifications or changes can be made according to the description of the present disclosure. The features, structures, methods, and other features of the exemplary embodiments described in the present disclosure can be combined in various ways to obtain additional and/or alternative exemplary embodiments. However, these changes and modifications will not deviate from the scope of the present disclosure.

FIG. 2 is a schematic diagram illustrating an exemplary computing device on which at least a portion of the medical system may be implemented according to some embodiments of the present disclosure. As shown in FIG. 2, a computing device 200 may include a processor 210, a memory 220, an input/output (I/O) 230, and a communication port 240.

The processor 210 may execute computer instructions (e.g., program codes) and perform functions of the processing device 140 according to techniques described in the present disclosure. The computer instructions may include, for example, routines, programs, objects, components, data structures, procedures, modules, and functions that perform specific functions described in the present disclosure. For example, the processor 210 may process data or information obtained from the medical device 110, the storage device 150, the terminal 130, and/or any other component of the medical system 100. In some embodiments, the processor 210 may include one or more hardware processors, such as a microcontroller, a microprocessor, a reduced instruction set computer (RISC), an application specific integrated circuit (ASIC), an application specific instruction set processor (ASIP), a central processing unit (CPU), a graphics processing unit (GPU), a physical processing unit (PPU), a microcontroller unit, a digital signal processor (DSP), a field programmable gate array (FPGA), an advanced RISC machine (ARM), a programmable logic device (PLD), any circuit and processor capable of performing one or more functions, or the like, or any combination thereof.

For illustration only, only one processor is described in the computing device 200. However, it should be noted that the computing device 200 disclosed in the present disclosure may also include a plurality of processors. Therefore, operations and/or method steps performed by one processor disclosed in the present disclosure may also be performed jointly or separately by a plurality of processors. For example, if the processor of the computing device 200 performs operations A and B in the present disclosure, it should be understood that the operations A and B may also be performed (for example, a first processor performs the operation A, a second processor performs the operation B, or the first processor and the second processor jointly perform operations A and B) jointly or separately by two or more different processors in the computing device 200.

The memory 220 may store data/information obtained from the medical device 110, the storage device 150, the terminal 130, and/or any other component of the medical system 100. In some embodiments, the memory 220 may include a mass memory, a removable memory, a volatile read-write memory, a read-only memory, or the like, or any combination thereof. For example, the mass storage may include a magnetic disk, an optical disk, a solid state drive, or the like. The removable memory may include a flash drive, a floppy disk, an optical disk, a memory card, a compressed disk, a magnetic tape, or the like. The volatile read-write memory may include a random access memory (RAM). The RAM may include a dynamic random access memory (DRAM), a double data rate synchronous dynamic random access memory (DDR SDRAM), a static random access memory (SRAM), a thyristor random access memory (T-RAM), a zero capacitance random access memory (Z-RAM), or the like. The ROM may include a mask ROM (MROM), a programmable ROM (PROM), an erasable programmable ROM (EPROM), an electrically erasable programmable ROM (EEPROM), a compact disc ROM (CD-ROM), a digital versatile disc ROM, or the like. In some embodiments, the memory 220 may store one or more programs and/or instructions to perform the exemplary methods described in the present disclosure.

The I/O 230 may input and/or output signals, data, information, or the like. In some embodiments, the I/O 230 may enable a user to interact with the processing device 140. In some embodiments, the I/O 230 may include an input device and an output device. The exemplary input device may include a keyboard, a mouse, a touch screen, a microphone, or the like, or any combination thereof. The exemplary output device may include a display device, a speaker, a printer, a projector, or the like, or any combination thereof. The exemplary display device may include a liquid crystal display (LCD), a light emitting diode (LED)-based display, a flat panel display, a curved screen, a television device, a cathode ray tube (CRT), a touch screen, or the like, or any combination thereof.

The communication port 240 may be connected to a network (e.g., the network 120) to facilitate data communication. The communication port 240 may establish a connection between the processing device 140, the medical device 110, the storage device 150, and/or the terminal 130. The connection may be a wired connection, a wireless connection, any other communication connection that may realize data transmission and/or reception, and/or a combination of the above connections. The wired connection may include, for example, a cable, an optical cable, a telephone line, or the like, or any combination thereof. The wireless connection may include, for example, Bluetooth, Wi-Fi, WiMAX, wireless local area network, ZigBee, mobile network (e.g., 3G, 4G, 5G), or the like, or any combination thereof. In some embodiments, the communication port 240 may be and/or include a standardized communication port, such as RS232, RS485, etc. In some embodiments, the communication port 240 may be a specially designed communication port. For example, the communication port 240 may be designed according to a digital imaging and communication in medicine (DICOM) protocol.

In some embodiments, a computer program may be stored in the memory 220 in the computing device 200, and the processor 210 may execute the computer program to realize one or more of a method for determining a time-activity curve, a method for processing image data, a method for determining kinetic parameters, and a method for determining an input function.

FIG. 3 is a flowchart illustrating a process for determining a time-activity curve according to the present invention.

In some embodiments, one or more operations for determining the time-activity curve may be performed by the processing device 140 in FIG. 1. The process 300 includes the following operations.

In operation 310, a plurality of dynamic frame images is obtained. In some embodiments, the operation 310 may be performed by the dynamic image obtaining module 510.

In some embodiments, the plurality of dynamic frame images may be a plurality of dynamic frame images scanned by the medical device 110 in FIG. 1. Each of the plurality of dynamic frame images includes dynamic image information of a target region, the plurality of dynamic frame images may provide tracer distribution image information at continuous time points (a plurality of frames), and reveal a variation of a tracer activity with time. The functional parameters of tissues and organs may be further obtained by applying the kinetic model to the dynamic images. The target region is a head region or an abdomen region. In further non-claimed examples, the target region may be a whole-body or a partial region of a body, such as an aortic region, a heart region, or the like.

In some embodiments, the plurality of dynamic frame images may be long axis dynamic images obtained by a long axis device or short axis dynamic images obtained by a short axis device, wherein the long axis dynamic images may include dynamic images of the whole-body or most of the body, and the short axis dynamic images may include dynamic images of partial region(s) of the whole-body.

In some embodiments, if the plurality of dynamic frame images are the long axis dynamic images, the operation 310 may further include: obtaining the long axis dynamic images including the target region; and/or segmenting the long axis dynamic images based on a range of the target region to obtain the plurality of dynamic frame images.

In some embodiments, the segmentation of the long axis dynamic images may be performed according to the range of the target region. For example, if the target region is the heart region, the long axis dynamic images may be segmented into dynamic images including a heart region as the plurality of dynamic frame images.

In some embodiments, if the plurality of dynamic frame images are the short axis dynamic images, the operation 310 may further include: obtaining the short axis dynamic images including the target region; and/or performing frame division and reconstruction on the short axis dynamic images to obtain the plurality of dynamic frame images.

In some embodiments, when scanning with the short axis device, a plurality of beds may usually be scanned correspondingly, and the plurality of dynamic frame images may be obtained through the frame division and reconstruction. More descriptions of the frame division and reconstruction may be found in the international application No. PCT/CN2021/099887, filed on June 11, 2021, entitled "SYSTEMS AND METHODS OF PARAMETRIC IMAGING". It should be noted that in some embodiments, according to an actual situation, the frame division and reconstruction may also be performed on the long axis dynamic images, and the short axis dynamic image may also be segmented. The present description is merely provided for the purposes of illustration, and not intended to limit the scope of the present disclosure.

In some embodiments, the plurality of dynamic frame images may also be motion-corrected dynamic images to correct an image error caused by a movement of a device or a scanned subject during a scanning process and ensure accuracy of the plurality of dynamic frame images.

In some embodiments, taking a scanning of a short axis PET imaging device as an example, the plurality of dynamic frame images may be dynamic images of the head obtained by performing the frame division and reconstruction. In some embodiments, the short axis PET imaging device may perform a whole-body dynamic scanning. For example, if the target region of scanning is the heart, the short axis PET imaging device may first perform a cardiac single bed scanning, and then perform a multi-passes unidirectional or multi-directional scanning of the whole-body (one scanning from head to feet or feet to head is one pass), and obtain dynamic images of the heart by performing the frame division and reconstruction as the plurality of dynamic frame images.

In operation 320, the time-activity curve is determined by inputting the plurality of dynamic frame images to a trained extraction model. In some embodiments, the operation 320 may be performed by the time-activity curve obtaining module 520.

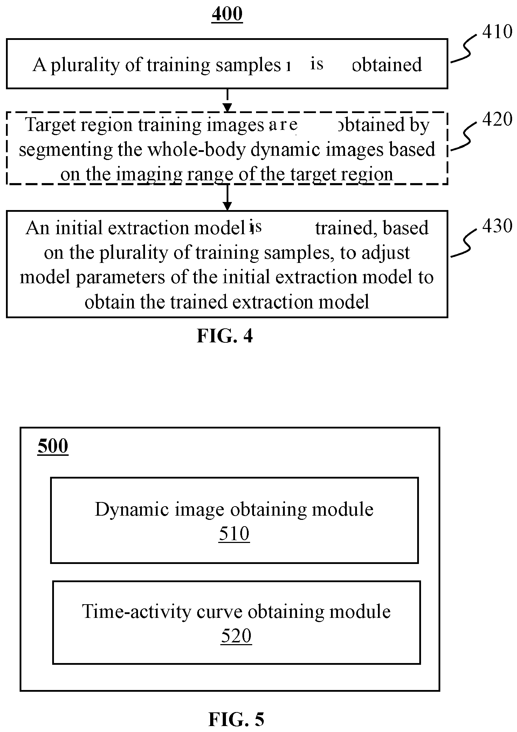

In some embodiments, the trained extraction model may be a machine learning model. In some embodiments, the machine learning model may include but not limited to a neural network model (e.g., a Convolutional Neural Network (CNN) model, a Deep Neural Network (DNN) model, a Recurrent Neural Network (RNN) model), a Support Vector Machine model, a Gradient Boosting Decision Tree (GBDT) model, or the like. More descriptions of a training process of the machine learning model may be found in the descriptions of FIG. 4.

In some embodiments, based on the example in the operation 310, an accurate time-activity curve may be obtained regardless of whether the plurality of dynamic frame images input to the trained extraction model are the dynamic images of the head or the dynamic images of the heart.

In some embodiments, after determining the time-activity curve, a dynamic parametric image may be further reconstructed according to actual needs. Therefore, in some embodiments, the process 300 may further include operation 330. In operation 330, a dynamic parametric image may be reconstructed based on the plurality of dynamic frame images and the time-activity curve.

The dynamic parametric image may reflect the metabolic information of the tracer in the target region, and the dynamic parametric image may be reconstructed based on the plurality of dynamic frame images and the time-activity curve, so as to obtain a more accurate result.

In some embodiments, the plurality of dynamic frame images may be input to the trained extraction model, and the trained extraction model may output the time-activity curve. More descriptions of the time-activity curve may be found elsewhere in the present disclosure, and details are not repeated here. In some embodiments, by establishing a direct mapping between the plurality of dynamic frame images and the time-activity curve, it is unnecessary to draw the region of interest, avoiding errors in the drawing process, and ensuring the accuracy of the time-activity curve.

FIG. 4 is a flowchart illustrating a process for training an extraction model according to the present invention. The trained extraction model in the operation 320 is obtained by training an initial machine learning model. The training process 400 includes: obtaining a plurality of training samples each of which including a sample dynamic image and a label of the sample dynamic image, the label including a time-activity curve corresponding to the sample dynamic image; and training an initial extraction model, based on the plurality of training samples, to adjust model parameters of the initial extraction model to obtain the trained extraction model.

In some embodiments, the sample dynamic image(s) used to train the initial extraction model may include one or more whole-body dynamic images. In some embodiments, the sample dynamic image(s) used to train the initial extraction model may also include one or more non-whole body dynamic image(s) (e.g., dynamic image(s) including an upper body, dynamic image(s) excluding the limbs, etc.). A sample dynamic image is obtained by segmenting the whole-body dynamic image based on an imaging range of the target region. Specifically, the imaging range of the target region may be determined based on a manual drawing or a model, and the whole-body dynamic images may be segmented based on the imaging range to obtain target images, so as to facilitate the training of the initial extraction model using the target images as the sample dynamic images.

The training process 400 using the whole-body dynamic images as the sample dynamic images includes the following operations.

In operation 410, a plurality of training samples is obtained. In some embodiments, the training samples may include whole-body dynamic images as training data, and time-activity curves corresponding to the whole-body dynamic images as labels. In some embodiments, a whole-body dynamic image refers to a dynamic image obtained by scanning the whole-body or most of the body, for example, the whole-body dynamic image may be scanned by a PET imaging device with an axial field of view of 2m long. It should be noted that in some embodiments, the whole-body dynamic images may also be obtained by other methods, which are not limited in the present disclosure.

In some embodiments, the whole-body dynamic images are acquired by whole-body simultaneous imaging. By using the whole-body simultaneous imaging, a corresponding time-activity curve may be used to establish real-time and continuous correspondence for all positions of the whole-body in a whole-body dynamic image, so that the trained extraction model has higher accuracy.

In some embodiments, the time-activity curve corresponding to the whole-body dynamic image may be obtained by other methods. For example, an accurate region of interest may be drawn by an experienced operator on the dynamic image, and a time-activity curve may be obtained based on the region of interest. In some embodiments, motion correction and/or partial volume correction may be performed before drawing to ensure the accuracy of the obtained time-activity curve.

In some non-claimed examples, the time-activity curve corresponding to the whole-body dynamic image may be obtained by collecting blood samples when the whole-body dynamic image is acquired; and detecting the blood samples to obtain the time-activity curve. As the reference standard of time-activity curve, the method of continuous collecting blood samples to obtain a concentration may make the label more accurate in the training process, thus ensuring the accuracy and effect of the trained extraction model. It should be noted that in some embodiments, it is also possible to acquire an accurate time-activity curve corresponding to the whole-body dynamic image by acquiring the dynamic image and performing necessary image correction (e.g., a motion correction, a partial volume correction, etc.) on the dynamic image, and use the time-activity curve as the label of training data.

The process 400 further includes operation 420. In operation 420, target region training images are obtained by segmenting the whole-body dynamic images based on the imaging range of the target region.

In some non-claimed examples, if the initial extraction model is trained with the whole-body dynamic images as the training data, in order to ensure the effect of model execution, a target region of an input image may correspond to the whole-body. However, in some embodiments, as in the above example of head scanning, the target region of the dynamic images is the head region. The whole-body dynamic images are segmented to obtain target region training images corresponding to the target region by performing the operation 420, that is, the whole-body dynamic images are segmented to obtain the head dynamic images, and the head dynamic images are used as the target region training images. In further non-claimed examples, if the target region of the dynamic image is the heart, the whole-body dynamic images may be segmented to obtain the cardiac dynamic images, and the cardiac dynamic images may be used as the target region training images.

In operation 430, an initial extraction model is trained, based on the plurality of training samples, to adjust model parameters of the initial extraction model to obtain the trained extraction model.

In some embodiments, according to different target regions, different training samples may be selected to train the initial extraction model to obtain a predicted value output by the initial extraction model, and the parameters of the initial extraction model may be adjusted based on the difference between the predicted value and the label until the number or count of iterations reaches a preset number or the initial extraction model converges, the training may be terminated.

In the present invention, the training data includes dynamic image(s) of a target region corresponding to a head region or an abdomen region, the label(s) are the time-activity curve(s) corresponding to the whole-body dynamic image(s), and the label(s) are obtained in a specific blood pool region corresponding to the aorta or the heart. The method for obtaining the label(s) may avoid the need for an additional partial volume correction or low accuracy of the time-activity curve(s) due to poor imaging conditions of the blood pool region (e.g., the head region) or small coverage of short axis imaging.

In some embodiments, the trained extraction model may include a classification sub-model and a plurality of extraction sub-models. The classification sub-model may be used to determine the target region included in the plurality of dynamic frame images. The plurality of extraction sub-models may be respectively used to input dynamic images of different target regions to obtain the time-activity curve.

In some embodiments, the training process of any sub-model may be similar to the training process of the initial extraction model illustrated in the process 400. The classification sub-model may be a classifier obtained by a machine learning process. When the classification sub-model inputs the plurality of dynamic frame images, the classification sub-model may output the target region(s) contained in the plurality of dynamic frame images.

In some embodiments, different extraction sub-models may correspond to different target regions, and the different extraction sub-models may be integrated into the trained extraction model, so that in actual execution, no matter whether the obtained plurality of dynamic frame images include any target region, appropriate sub-models may be automatically matched to obtain the time-activity curve.

It should be noted that the above descriptions of the processes 300 and 400 are only for example and explanation, and do not limit the scope of the present disclosure. For those skilled in the art, various modifications and changes can be made to the processes 300 and 400 under the guidance of the present disclosure. However, these modifications and changes are still within the scope of the present disclosure. For example, the operation 420 may not be executed, and the operation 430 may be executed directly after the operation 410.

FIG. 5 is a schematic diagram illustrating an exemplary system for determining the time-activity curve according to some embodiments of the present disclosure. In some embodiments, the time-activity curve extraction system 500 may include a dynamic image obtaining module 510 and a time-activity curve obtaining module 520.

The dynamic image obtaining module 510 may be configured to obtain the plurality of dynamic frame images. Each of the plurality of dynamic frame images may at least include dynamic image information of a target region.

In some embodiments, more descriptions about the plurality of dynamic frame images can be found elsewhere (e.g., the operation 310 and its descriptions) in the present disclosure. In some embodiments, the dynamic image obtaining module 510 may obtain data and/or information related to the medical system 100 from one or more other components (e.g., the medical device 110, the storage device 150) of the medical system 100.

The time-activity curve obtaining module 520 may be configured to input the plurality of dynamic frame images to obtain the time-activity curve.

In some embodiments, more descriptions of the trained extraction model can be found elsewhere (e.g., the operation 320, the process 400, and descriptions thereof) in the present disclosure. In some embodiments, the time-activity curve obtaining module 520 may obtain data and/or information related to the medical system 100 from one or more other components (e.g., the medical device 110, the storage device 150) of the medical system 100.

Beneficial effects that may be brought by the method for determining the time-activity curve may include but are not limited to: (1) by establishing the direct mapping between the dynamic images and the time-activity curve, it is not necessary to draw the region of interest in the dynamic images, which avoids the errors generated in the drawing process and ensures the accuracy of the time-activity curve; (2) the labels used in the training are the time-activity curve(s) corresponding to the whole-body dynamic image(s), so that the trained extraction model does not need to perform the additional partial volume correction, the accuracy of the time-activity curve can be improved, and the trained extraction model has good accuracy when an input is a short axis scanning image.

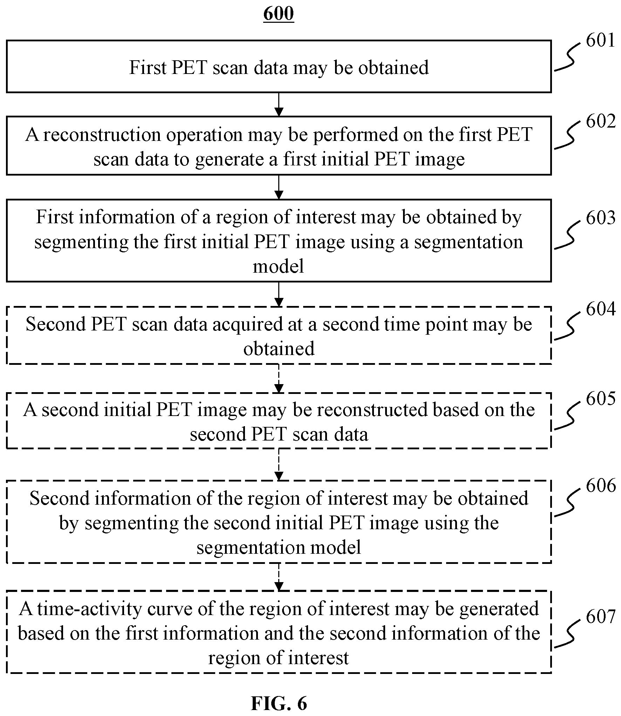

As shown in FIG. 6, in some embodiments, some embodiments of the present disclosure may provide a method for determining an input function. One or more operations of the process 600 may be performed by the processing device 140 in FIG. 1. The process 600 may include one or more of the following operations.

In operation 601, first PET scan data may be obtained. The first PET scan data may be acquired by a PET device (e.g., the medical device 110 in FIG. 1) at a first time point. In some embodiments, the first PET scan data may include kinetic parameters that reflect biological characteristics of a local scanned tissue or organ.

In operation 602, a reconstruction operation may be performed on the first PET scan data to generate a first initial PET image. The reconstruction operation may include obtaining an initial PET image based on a specific algorithm or process based on PET scan data and/or other data. For example, a reconstruction parameter curve may be constructed based on the first PET scan data and values of corresponding kinetic parameters, and the first initial PET image may be reconstructed using the constructed reconstruction parameter curve.

In operation 603, first information of a region of interest may be obtained by segmenting the first initial PET image using a segmentation model.

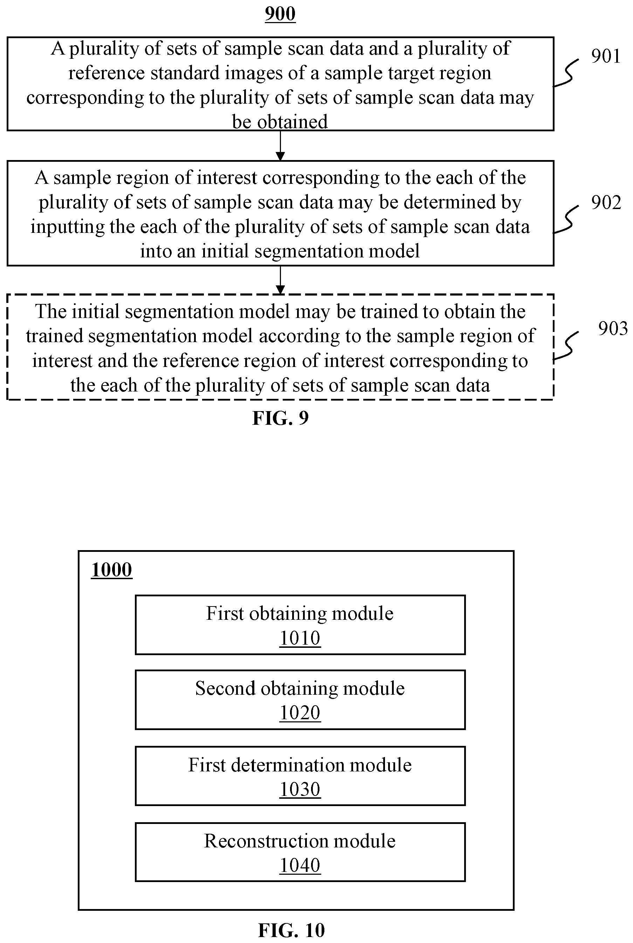

In some embodiments, the segmentation model may be a machine learning model. In some embodiments, the machine learning model may include but not limited to a neural network model (e.g., a CNN model, a DNN model, a RNN model), a support vector machine model, a GBDT model, or the like. In some embodiments, the segmentation model may include a generative adversarial network (GAN) model. More descriptions of the training of the segmentation model may be found elsewhere in the present disclosure (e.g., FIG. 9 and descriptions thereof).

In some embodiments, the region of interest may be a part of a PET scanning region. After selecting the region of interest, further analysis or image reconstruction may be performed based on parameters relating to the region of interest. For example, if a scanning target region is a brain region, the region of interest may be a brain stem region of the brain or other regions of the brain, or the like, which is not limited here. Optionally, in some embodiments, the region of interest may include an aortic region.

In some embodiments, parameters and structure data files of the segmentation model may be processed and stored in a memory (e.g., the memory 220 in FIG. 2) of a processing device. When the operation 603 is executed, the first information of the region of interest may be obtained by calling a network interface to obtain the parameters and the structure data files of the segmentation model, and segmenting the first initial PET image using the segmentation model.

In some embodiments, in the operation 603, the processing device may segment the first initial PET image using the segmentation model within a preset time after the reconstruction operation. In some embodiments, the segmentation may be performed immediately after the first initial PET image is obtained, or may be performed within a period of time (e.g., less than five minutes, less than twenty minutes, etc.), or may be performed after obtaining a plurality of initial PET images (e.g., obtaining two initial PET images or obtaining more than three initial PET images).

In some embodiments, the first information of the region of interest may include PET image information of the region of interest and/or mask image information of the region of interest. The mask image may be a binary image (including pixels with pixel values of 0 and/or 1) with a same size as the PET image. When the mask image is used in image processing, the region including pixels with pixel values of 1 may be processed, and the region including pixels with pixel values of 0 (or a mask region) may not be processed. In some embodiments, the first initial PET image may be segmented using the segmentation model to obtain PET image information of the region of interest, and the mask image information of the corresponding region of interest may be obtained based on the PET image information of the region of interest. The mask image information of the region of interest may occupy little space and do not include privacy scanning information, which is convenient for subsequent use or sharing with a third party.

In some embodiments, the processing device may, in response to an instruction, segment the first initial PET image using the segmentation model within the preset time after the reconstruction operation to obtain the first information of the region of interest.

In some embodiments, the instruction may be used to instruct the processing device to obtain the first information of the region of interest, including the PET image information of the region of interest and the mask image information of the region of interest. It should be noted that in some other embodiments, the processing device may be configured to obtain only the PET image information of the region of interest according to a default setting when no instruction is received. In addition, in some embodiments, the processing device may obtain only the PET image information of the region of interest in response to the instruction, and obtain the PET image information of the region of interest and the mask image information of the region of interest according to a default setting when the instruction is not accepted.

In some embodiments, the first information of the region of interest may be stored in a DICOM format. For example, the mask image information of the region of interest may be saved as a segmentation file in the DICOM format for offline parametric imaging analysis or reading and display of third-party workstation software. In some embodiments, the method for processing image data may also include reconstructing and/or segmenting based on PET scan data to obtain the time-activity curve of the region of interest. The method may further include one or more of the following operations.

In operation 604, second PET scan data acquired at a second time point may be obtained.

The second time point may be a certain time point before or after the first time point. In some embodiments, an interval between the first time point and the second time point may be controlled not to exceed a preset threshold, such as no more than 10 seconds, 20 seconds, or 1 minute. A scanned tissue or organ in the second PET scan data may be the same as the scanned tissue or organ in the first PET scan data and may include kinetic parameters that reflect the biological characteristics of the local scanned tissue or organ.

In operation 605, a second initial PET image may be reconstructed based on the second PET scan data. An operation of generating the second initial PET image by reconstructing based on the second PET scan data may be similar to the operation of generating the first initial PET image. More descriptions of the reconstruction may be found elsewhere in the present disclosure (e.g., operation 602 and descriptions thereof).

In operation 606, second information of the region of interest may be obtained by segmenting the second initial PET image using the segmentation model.

If the scanned tissue or organ in the second PET scan data is the same as the scanned tissue or organ in the first PET scan data, the region of interest in the second PET scan data is also the same as the region of interest in the first PET scan data. A process of obtaining the second information of the region of interest by segmenting the second initial PET image using the segmentation model may be similar to the process of obtaining the first information of the region of interest. More descriptions may be found elsewhere in the present disclosure (e.g., operation 603 and descriptions thereof).

In operation 607, a time-activity curve of the region of interest may be generated based on the first information and the second information of the region of interest.

The time-activity curve may be used to characterize a parameter change of a drug over time in the region of interest. In some embodiments, the processing device may determine a time activity of each voxel in the region of interest based on the first information and the second information of the region of interest, and then draw the time activity of the each voxel in the region of interest to obtain the time-activity curve in the region of interest.

In some embodiments, the method for processing image data may further include: reconstructing a dynamic parametric image based on the first initial PET image, the second initial PET image, and the time-activity curve. The dynamic parametric image may reflect the metabolic information of the tracer in the target region. The dynamic parametric image may be reconstructed based on the PET image and the time-activity curve and may be used for further reference or analysis.

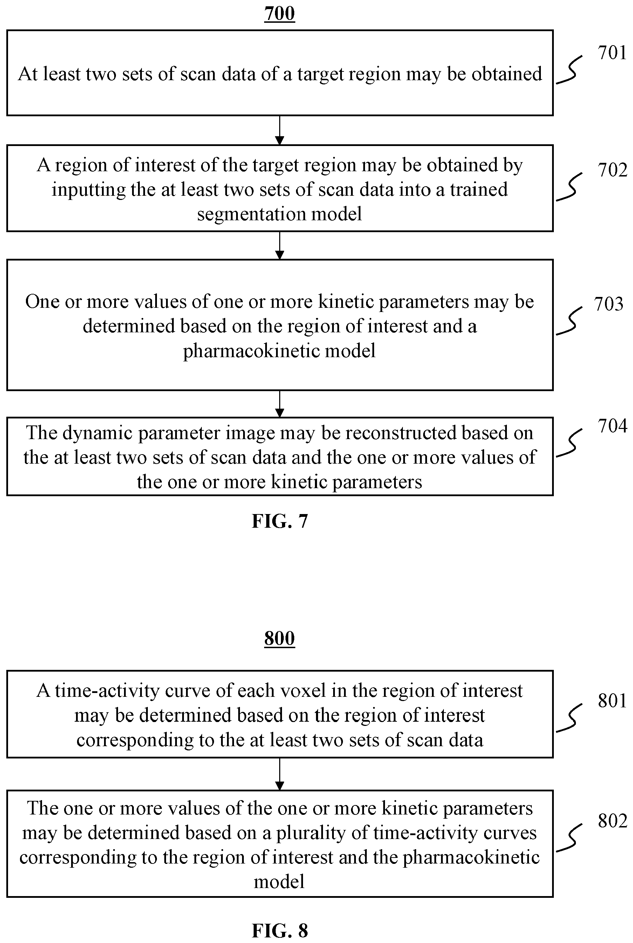

In some embodiments, as shown in FIG. 7, a process for determining a kinetic parametric image may be provided. One or more operations of the process 700 may be performed by the processing device 140 in FIG. 1. In some embodiments, the process 700 may include one or more of the following operations.

In 701, at least two sets of scan data of a target region may be obtained. In some embodiments, time information corresponding to the at least two sets of scan data may be different.

The target region may be a partial region of an object or a whole-body region of the object. The object may be an animal body, a human body, a phantom, etc. The partial region of the object may be the brain, legs, chest, abdomen, etc., of the object. The scan data may include scan data obtained by a positron emission tomography device or a single photon emission tomography device. For example, the scan data may be the PET data. It should be noted that when the above-mentioned scan data is PET data, it is possible to avoid obtaining a user's CT image, thereby reducing harm of a CT scanning radiation dose to the human body. In some embodiments, the at least two sets of scan data obtained by the processing device may be scan data of the legs obtained at different times or the scan data of the abdomen obtained at different times. In some embodiments, the processing device may obtain the at least two sets of scan data of the target region from a Picture Archiving and Communication Systems (PACS) server, or may obtain the at least two sets of scan data of the target region in real-time from a scanning device. For example, the processing device may obtain the at least two sets of scan data of the target region in real-time from a PET device. In some embodiments, the at least two sets of scan data of the target region may be obtained in real-time from a CT device. As an example, the obtained at least two sets of scan data of the target region may include the scan data of the target region obtained at a first time, the scan data of the target region obtained at a second time, and so on, wherein the second time may be later or earlier than the first time.

In 702, a region of interest of the target region may be obtained by inputting the at least two sets of scan data into a trained segmentation model.

Specifically, the processing device may input the obtained at least two sets of scan data into the trained segmentation model to obtain the region of interest of the target region. It can be understood that the trained segmentation model may be a model trained by using historical scan data. The trained segmentation model may be a model for analyzing CT scan data or a model for analyzing PET scan data. The trained segmentation model may be a Long Short-Term Memory (LSTM) model or any other type of neural network model. The processing device may sequentially input the obtained at least two sets of scan data into the trained segmentation model, or may integrate the obtained at least two sets of scan data and input the integrated at least two sets of scan data into the trained segmentation model.

In 703, one or more values of one or more kinetic parameters may be determined based on the region of interest and a pharmacokinetic model. In some embodiments, the one or more kinetic parameters may be related to metabolic information of a tracer in the target region.

Pharmacokinetics can reflect a change of drug content in a subject over time. Pharmacokinetics may be used to study absorption, distribution, metabolism and excretion of a drug in the subject. The pharmacokinetic model may be used to analyze the obtained regions of interest and measure a curve of drug concentration in each organ over time (i.e., the time-activity curve (TAC)). Specifically, in some embodiments, the determined region of interest may be analyzed by the pharmacokinetic model to determine the kinetic parameters related to the metabolic information of the tracer in the target region. In some embodiments, the processing device may solve the pharmacokinetic model and determine the value(s) of the kinetic parameter(s) based on the determined region of interest in the scan data, or the processing device may obtain relevant parameters of the kinetic model and determine the value(s) of the above kinetic parameter(s), for example, an input function (IF) and/or a time-activity curve (TAC) of the region of interest. The input function may reflect a change of a tracer concentration in plasma with time. For example, the input function may indicate the change of the tracer concentration in plasma, and the TAC of the region of interest may reflect the change of the tracer concentration in the region of interest with time. In some embodiments, the input function may be expressed as the TAC. In some embodiments, the pharmacokinetic model may include a compartment model (for example, a one compartment model and a two compartment model) or a retention model.

In the above process for determining kinetic parameters, by obtaining the at least two sets of scan data with different time information of the target region and inputting the two sets of different scan data into the trained segmentation model, the region of interest of the target region can be quickly obtained, so that the value(s) of the kinetic parameter(s) can be quickly determined based on the determined region of interest and the pharmacokinetic model. The kinetic parameter(s) may be related to the metabolic information of the tracer in the determined target region.

In some embodiments, as shown in FIG. 8, the operation 703 may be performed according to the process 800. The process 800 may include one or more of the following operations.

In 801, a time-activity curve of each voxel in the region of interest may be determined based on the region of interest corresponding to the at least two sets of scan data.

The time-activity curve of the each voxel in the region of interest of the scan data may be used to characterize the parameter change of the drug with time in the each voxel in the region of interest. In some embodiments, the processing device may determine the time activity of the each voxel in the region of interest based on the regions of interest of the at least two sets of scan data, and then draw the time activity of the each voxel in the region of interest to obtain the time-activity curve of the each voxel in the region of interest of the scan data.

In 802, the one or more values of the one or more kinetic parameters may be determined based on a plurality of time-activity curves corresponding to the region of interest and the pharmacokinetic model.

In some embodiments, the processing device may solve the pharmacokinetic model based on the time-activity curve of the each voxel in the region of interest to obtain the value(s) of the kinetic parameter(s) related to the metabolic information of the tracer in the determined target region. For example, the processing device may input the time-activity curve of the each voxel in the region of interest into the pharmacokinetic model to obtain the value(s) of the kinetic parameter(s), or the processing device may obtain the time activity of the each voxel based on the time-activity curve of the each voxel in the region of interest, and then substitute the time activity of the each voxel into the pharmacokinetic model to determine the value(s) of the kinetic parameter(s).

In some embodiments, the processing device can quickly determine the time-activity curve of the each voxel in the region of interest based on the region of interest of the at least two set of scan data of the obtained target region, so that the value(s) of the kinetic parameter(s) can be quickly determined based on the obtained time-activity curve and the pharmacokinetic model. In some embodiments, the value(s) of kinetic parameter(s) can be accurately determined based on the time-activity curve and the pharmacokinetic model, which improves the accuracy of the value(s) of the kinetic parameter(s).

As illustrated above, the at least two sets of scan data may be input into the trained segmentation model to obtain the region of interest of the at least two sets of scan data. In some embodiments, it is necessary to train an initial segmentation model to obtain the trained segmentation model. In some embodiments, as shown in FIG. 9, the training process may include one or more of the following operations.

In 901, a plurality of sets of sample scan data and a plurality of reference standard images of a sample target region corresponding to the plurality of sets of sample scan data may be obtained. A reference region of interest corresponding to each of the plurality of sets of sample scan data may be marked in a corresponding one of the plurality of reference standard images.

The sample scan data may include scan data obtained at different times. In some embodiments, the sample scan data may include PET image data or CT image data. In some embodiments, the processing device may draw a same target region corresponding to the sample scan data to obtain a reference standard image of the same target region corresponding to the sample scan data, that is, the region of interest of the sample scan data may be marked in the obtained reference standard image. In some embodiments, the reference standard image may be an image with structural anatomical information, such as a CT image or an MR image. In the present invention, the target region is a head region or an abdomen region of a scanning object. In some embodiments, the region of interest of the sample scan data may be marked in the reference standard image of the same target region corresponding to the sample scan data may be any region in the target region. For example, if the target region is a brain region, the region of interest may be a brain stem region, or any other brain region, etc.

In 902, a sample region of interest corresponding to the each of the plurality of sets of sample scan data may be determined by inputting the each of the plurality of sets of sample scan data into an initial segmentation model.

In some embodiments, the processing device may input the obtained sample scan data into the initial segmentation model, and determine the sample region of interest in the sample scan data through the initial segmentation model. For example, the processing device may input head scan data of the scanning object into the initial segmentation model to determine the sample region of interest in the head scan data, or the processing device may input abdominal scan data of the scanning object into the initial segmentation model to determine the sample region of interest in the abdominal scan data. In some embodiments, the initial segmentation model may be or include a machine learning model, such as a deep learning network model, which may automatically learn a large number of parameters obtained from the sample scan data and the reference standard images corresponding to the sample scan data, the large number of parameters may be used to identify and quantify the sample scan data corresponding to the region of interest of the reference standard images, which can greatly accelerate an obtaining speed of the region of interest. In some embodiments, the deep learning network model may include a generative adversarial network model. In some embodiments, the problem of a small amount of sample image data can be solved by generating the generative adversarial network model to train the sample image data to increase a count of samples. The generative adversarial network model may include a generation network model and a discrimination network model. The sample scan data may be input to the generation network model, and the output result may need to imitate the region of interest in the reference standard image corresponding to the sample scan data as much as possible. The input of the discrimination (network) model may be the output of the generation (network) model. The purpose is to distinguish the output of the generation model from the sample scan data as much as possible, and the generation model should generate the region of interest of the sample scan data that the discrimination model can't identify as true or false as much as possible. The two models may antagonize each other and parameters may be constantly adjusted. The ultimate purpose may be to make the discrimination network model unable to judge whether the output of the generation network model is true. That is, based on the generative adversarial network model, the region of interest of the sample scan data that is similar to the region of interest of the reference standard image and determined to be true by the discrimination network may be generated, and accuracy of determining the region of interest from the sample scan data can be effectively improved.

In some embodiments, if the sample scan data includes PET data or SPECT data, compared with image data with structural information such as CT image data, the sample scan data may be preprocessed. For example, an image enhancement processing may be performed on the sample scan data, before the sample scan data is input into the initial segmentation model, so that an outline of the region of interest can be clearer. In some embodiments, before the sample scan data is input into the initial segmentation model, the image(s) corresponding to the sample scan data may be segmented, for example, a background image region of a non-interest region may be segmented off to retain the region of interest. By preprocessing the sample scan data, the accuracy of determining the region of interest using the initial segmentation model can be improved. In some embodiments, in order to improve the accuracy of determining the region of interest, in the process of training the initial segmentation model, an outline of a traditional CT image may be mapped to a PET image, a pair of images (i.e., the outline of the traditional CT image, and that mapped to the PET image) may be taken as label data, and the sample scan data may be input into the initial segmentation model to obtain the sample region of interest of the sample scan data. The initial segmentation model may be trained by using the label data and the obtained sample region of interest of the sample scan data. In some embodiments, considering limitations of hardware performance on a training process during the training of the initial segmentation model, for example, considering that a capacity of a graphics processing unit (GPU) of a processing device for training the initial segmentation model is limited, it is possible to divide the sample scan data into blocks and input the blocks into the initial segmentation model, so as to improve training efficiency of the initial segmentation model, and speed up the training of the initial segmentation model.

In some embodiments, a registration function of the sample scan data and a reference standard image corresponding to the sample scan data (i.e., the motion field(s) of the sample scan data and the reference standard image corresponding to the sample scan data) may be determined by the initial analytical model. Generally, when scanning a patient, for example, a movement of organ positions such as a heart and a lung caused by drug stimulation and a movement of organ positions caused by respiratory movement and heartbeat movement may lead to different regions of interest corresponding to the sample scan data at different times, and scanning time of the sample scan data and the reference standard image may be different. The difference may lead to a difference between the sample scan data and the reference standard image. In some embodiments, according to the registration function obtained by the initial segmentation model, image registration may be performed on the sample scan data and the reference standard image corresponding to the sample scan data, so as to optimize the training of the initial segmentation model and improve accuracy of the region of interest for obtaining the sample scan data.

In 903, the initial segmentation model may be trained to obtain the trained segmentation model according to the sample region of interest and the reference region of interest corresponding to the each of the plurality of sets of sample scan data.

In some embodiments, the processing device may compare a sample region of interest with the reference region of interest corresponding to the each of the plurality of sets of sample scan data to obtain a loss function of the initial segmentation model, train the initial segmentation model by using the loss function of the initial segmentation model to obtain the above-mentioned trained segmentation model. In some embodiments, the processing device may adjust parameter(s) of the initial segmentation model by using a value of the loss function of the initial segmentation model, obtain the adjusted segmentation model by using the adjusted parameter(s) of the initial segmentation model, and further obtain the trained segmentation model according to the adjusted segmentation model.

In some embodiments, the processing device can quickly determine the sample region of interest of the sample scan data by obtaining the sample scan data and the reference standard image of the same target region corresponding to the sample scan data and inputting the sample scan data into the initial segmentation model, and then can quickly train the initial segmentation model according to the sample region of interest and the region of interest of the sample scan data, thereby improving efficiency of obtaining the trained segmentation model.

FIG. 10 is a schematic diagram illustrating an exemplary system for determining kinetic parameters according to some embodiments of the present disclosure. The system 1000 for determining kinetic parameters may include a first obtaining module 1010, a second obtaining module 1020, a first determination module 1030, and a reconstruction module 1040.

The first obtaining module 1010 may be configured to obtain the at least two sets of scan data of the target region. In some embodiments, time information corresponding to the at least two sets of scan data may be different. In some embodiments, the scan data may include scan data obtained by a positron emission tomography device or a single photon emission tomography device.

The second obtaining module 1020 may be configured to input the at least two sets of scan data into the trained segmentation model to obtain the region of interest of the target region. In some embodiments, the region of interest may include an aortic region.

The first determination module 1030 may be configured to determine the value(s) of the kinetic parameter(s) based on the region of interest and the pharmacokinetic model. In some embodiments, and the kinetic parameter(s) may be related to determining the metabolic information of the tracer in the target region. In some embodiments, the pharmacokinetic model may include a compartment model.

On the basis of the above embodiments, optionally, the first determination module 1030 may include a first determination unit and a second determination unit (not shown). The first determination unit may be configured to determine the time-activity curve of the each voxel in the region of interest based on the region of interest of the at least two sets of scan data. The second determination unit may be configured to determine the value(s) of the kinetic parameter(s) based on the time-activity curve and the pharmacokinetic model.

On the basis of the above embodiments, optionally, the system 1000 may further include a third obtaining module, a second determination module, and a training module (not shown). The third obtaining module may be configured to obtain the sample scan data and the reference standard image of the same target region corresponding to the sample scan data. The region of interest of the sample scan data may be marked in the reference standard image. The second determination module may be configured to input the sample scan data into the initial segmentation model to determine the sample region of interest of the sample scan data. The training module may be configured to train the initial segmentation model according to the sample region of interest and the region of interest of the sample scan data to obtain the trained segmentation model. In some embodiments, the initial segmentation model may include a generative adversarial network model.

The modules in the determination system of the above dynamic parameters can be achieved through the software, hardware, and its combination. The above modules may be embedded in or independent of a processor in the processing system in a form of hardware, or may be stored in a memory in the processing system in a form of software so that the processor can call and execute the operations corresponding to the above modules.

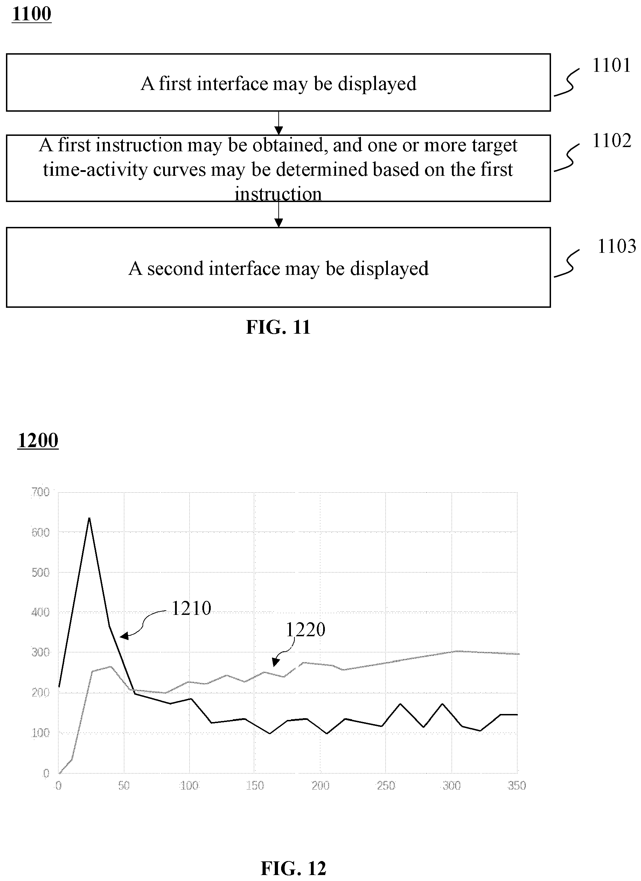

Referring to FIG. 12, for example, in FIG. 12, an abscissa of a starting point of a time-activity curve 1210 is close to 200, and a normal time-activity curve should start from an origin. It can be considered that the time-activity curve 1210 may have operational errors during the acquisition process, so it may be eliminated. The time-activity curve 1220 in FIG. 12 does not have a peak that is significantly higher than other points, so it may be eliminated.

The screening of the time-activity curve(s) with the obviously inconsistent shape(s) may be completed before a user determines the target time-activity curve(s) or may be performed after the user determines the target time-activity curve(s), there is no specific limitation here. In some embodiments, curves that are obviously inconsistent in shape may be eliminated based on other features of the target time-activity curves according to user requirements, there is no specific limitation here.

In some embodiments, the time-activity curves with obviously inconsistent shapes can be automatically eliminated without the need to require the user to confirm the correctness of the time-activity curves one by one or write a program to eliminate the curves, which improves the efficiency and accuracy of generating the target input function curves.

In some embodiments, the displaying the second interface may further include the following operations: determining an average peak time of the plurality of time-activity curves, and translating the plurality of time-activity curves based on the average peak time; performing a screening operation on the plurality of time-activity curves based on starting times of the plurality of translated time-activity curves, and determining one or more time-activity curves, among the plurality of translated time-activity curves, whose starting times are within a second preset range as target time-activity curve(s).

Exemplarily, the abscissas of the peaks of all the time-activity curves may be obtained, and an average value of the abscissas may be obtained to obtain an average abscissa, and the peaks of the time-activity curves may be translated to the average abscissa. It may be determined whether the abscissa of the starting point of each of the translated time-activity curves is negative. If the abscissa is negative, the corresponding translated time-activity curve may be eliminated.

It should be understood that in some embodiments, the abscissas of the starting points of the translated time-activity curves may be compared with coordinates of other points, there is no specific limitation here.

In some embodiments, the screening based on the starting time of the time-activity curve may further include the following operations: displaying a third interface, the third interface presenting a result of the screening operation.

In some embodiments, as shown in FIG. 11, a method for determining an input function may be provided. One or more operations of the process 1100 may be performed by the processing device 140 in FIG. 1. The process 1100 may include one or more of the following operations.

In operation 1101, a first interface may be displayed. The first interface may present a plurality of time-activity curves. The time-activity curve (TAC) is a curve that reflects a concentration of a radioactive tracer in a specific region (such as tissue or plasma). A Y axis of the time-activity curve may represent concentration and an X axis of the time-activity curve may represent time. In some embodiments, the time-activity curve may be directly or processed as an input function of the plasma (IF) of the kinetic model to facilitate a further analysis of drug metabolism or a solution of kinetic parameter, etc.

Merely by way of example, a plurality of time-activity curves displayed on the first interface may be a plurality of time-activity curves corresponding to a region of interest, and may specifically include the time-activity curves corresponding to the each voxel in the region of interest. It can be understood that the plurality of time-activity curves may be time-activity curves derived from historical data or time-activity curves generated in real-time, as long as time-activity curves may characterize a relationship between the concentration of the radioactive tracer and the time in the region of interest. There is no specific limitation here.

In some embodiments, the target time-activity curve may include at least one historical input function curve. The first interface can directly display the shape of multiple time-activity curves. It can also use the names of the time-activity curves to characterize different time-activity curves.

In some embodiments, the first interface may further include relevant information of a scanning object corresponding to the plurality of time-activity curves, so as to facilitate a user to select target time-activity curve(s) from the plurality of time-activity curves. For example, the relevant information of the scanning object may be displayed when a corresponding time-activity curve is selected, or the relevant information may be folded and clicked to be shown when a user needs to see the relevant information. In some embodiments, the relevant information of the scanning object may include file information of the scanning object, scanned part(s), scanning parameter(s), etc.

In some embodiments, the relevant information of the scanning object may include similarity information between the scanning object and a current object. In some embodiments, the similarity information between the scanning object and the current object may be determined based on a preset algorithm, or a model, or parameters such as a condition of the scanning object or a scanning result, the similarity information may be provided for reference when a user selects the time-activity curve. For example, when the user selects the time-activity curve, the user may be recommended to select a time-activity curve above a certain similarity threshold (such as 80% or 90%).

In some embodiments, the target time-activity curve(s) may be given corresponding weight(s) in a fitting operation based on the similarity information corresponding to the target time-activity curve(s). Specifically, the higher the similarity corresponding to the target time-activity curve, the higher the weight of the target time-activity curve in the fitting operation. Conversely, the lower the similarity corresponding to the target time-activity curve, the lower the weight of the target time-activity curve in the fitting operation. Therefore, the analysis value of the obtained target time-activity curve(s) is improved.

In some embodiments, at least one of the plurality of time-activity curves may be obtained by: obtaining the plurality of dynamic frame images, each of the plurality of dynamic frame images at least including dynamic image information of a target region; and/or determining at least one of the plurality of time-activity curves by inputting the plurality of dynamic frame images to the trained extraction model. More descriptions of the extraction model may be found elsewhere in the present disclosure (e.g., FIGs. 1-5 and descriptions thereof).

In some embodiments, the at least one of the plurality of time-activity curves may be obtained by: obtaining first positron emission tomography (PET) scan data acquired at a first time point and second PET scan data acquired at a second time point; performing a reconstruction operation on the first PET scan data and the second PET scan data to generate a first initial PET image and a second initial PET image, respectively; obtaining first information of a region of interest by segmenting the first initial PET image using a segmentation model; obtaining second information of the region of interest by segmenting the second initial PET image using the segmentation model; and/or generating the at least one time-activity curve based on the first information and the second information of the region of interest. More descriptions may be found elsewhere in the present disclosure (e.g., FIGs. 1-10 and descriptions thereof).

In some embodiments, the plurality of time-activity curves may include time-activity curve(s) uploaded to a preset system by user(s). In some embodiments, the preset system may include Picture Archiving and Communication Systems (PACS) described above or other systems, the preset system is not limited in the present disclosure.

In 1102, a first instruction may be obtained, and one or more target time-activity curves may be determined based on the first instruction. It can be understood that the first instruction may be for the user to select from the plurality of time-activity curves displayed on the first interface, select part or all of the plurality of time-activity curves, to generate an input function curve. For example, the first instruction may be an operation instruction to select the target time-activity curve(s) and confirm completion of the selection.

In 1103, a second interface may be displayed. In some embodiments, the second interface may present a target input function curve generated based on the one or more target time-activity curves. For example, after the user selects the target time-activity curve(s), the processing device may generate a corresponding target input function curve according to the target time-activity curve(s), and display the target input function curve in the second interface.

In some embodiments, the target input function curve may be obtained by performing the fitting operation to the target time-activity curve(s). In some embodiments, the target input function curve(s) may be fitted based on an automatic fitting function provided by software, or abscissa(s) and/or ordinate(s) of one or more points of the target input function curve(s) may be manually selected by an operator to achieve a fitting of the target input function curve(s).

It can be understood that based on the obtained target input function curve(s), a target input function curve matching actual needs may be obtained for analysis.

In some embodiments, if the first instruction is an operation instruction of selecting the target time-activity curve(s) and confirming completion of the selection, the corresponding target input function curve may be generated and displayed immediately after the user confirms the completion of the selection. The corresponding target input function curve may also be generated and displayed after the user confirms that the selection is completed and receives a generation instruction. It can be understood that a generation timing described above may be selected according to user needs, and there is no specific limitation here.

The above process for determining the input function, through displaying the first interface, the first interface presenting a plurality of time-activity curves; obtaining the first instruction, and determining one or more target time-activity curves from the plurality of time-activity curves based on the first instruction; and displaying the second interface, the second interface presenting a target input function curve generated based on the one or more target time-activity curves, actual data can be selected according to a user's needs and the corresponding input function curve may be generated for analysis, thereby accurately matching the actual needs and making analysis results more accurate.

In some embodiments, the displaying the second interface may further include the following operations: in response to the first instruction, performing a screening operation on the plurality of time-activity curves according to starting times of the plurality of time-activity curves, and determining a time-activity curve, among the plurality of time-activity curves, whose starting time is within a first preset range as a target time-activity curve. It can be understood that in a process of generating the target input function curve, a small part of the plurality of time-activity curves may have shape(s) that are obviously inconsistent with that of most of the plurality of time-activity curves. In order to make this small part of obviously inconsistent time-activity curves not affect a shape of a finally generated target input function curve, this small part of time-activity curves may be removed and not participate in the process of generating the target input function curve.

In some embodiments, the plurality of time-activity curves may be filtered according to a user's selection. In some embodiments, the method may include: obtaining a third instruction; before the fitting operation, performing, based on the third instruction, a screening operation on the one or more target time-activity curves to remove an undesired time-activity curve that dissatisfies a preset rule.

In some embodiments, an option (such as "filtering") may be preset in the operation interface, and the user may click the option and send a third instruction to eliminate the time-activity curve(s) that does not meet the preset rule. In some embodiments, the third instruction may be a clicked operation instruction.

In some embodiments, the preset rule may include that a starting time of each of the one or more target time-activity curves is within a first preset range and/or a starting time of each of the one or more target time-activity curves after translation according to an average peak time is within a second preset range.

In some embodiments, the plurality of time-activity curves may be automatically screened after being obtained. In some embodiments, the method may include: in response to the first instruction, performing a screening operation on the plurality of time-activity curves according to starting times of the plurality of time-activity curves, and determining a time-activity curve, among the plurality of time-activity curves, whose starting time is within a first preset range as a target time-activity curve.

Whether it is selected by the user or automatically by the software, it can be determined whether it needs to be eliminated according to a shape or value of the time-activity curve.

Specifically, one or more time-activity curves with obviously inconsistent shapes may be determined according to the starting time and eliminated. The time-activity curve whose starting time is within the preset range may be a shape-matched time-activity curve, which is determined as the target time-activity curve. It can be understood that in a coordinate system where the time-activity curve is located, the starting time may be an abscissa of a starting point of the time-activity curve. In some embodiments, whether the shape of the time-activity curve is obviously inconsistent may be determined based on the starting time of the time-activity curve, that is, the abscissa of the starting point of the time-activity curve. Specifically, the abscissa of the starting point of the time-activity curve may be compared with a position of a coordinate origin.

Specifically, first, the starting point of the time-activity curve may be obtained, and whether the abscissa of the starting point is negative may be determined. If the abscissa of the starting point is negative, the time-activity curve may be eliminated. It should be understood that in some embodiments, the abscissa of the starting point of the time-activity curve may be compared with coordinates of other points, which is not specifically limited here.

It should be noted that in some embodiments, image processing (e.g., determining a time-activity curve, reconstructing a dynamic parametric image, determining the value(s) of kinetic parameter(s)) as illustrated in FIGs. 3, 6, 7 and 8, interface displaying as illustrated in FIG. 11, and model training as illustrated in FIGs. 4 and 9 may be performed on a single processing device or separate processing devices.

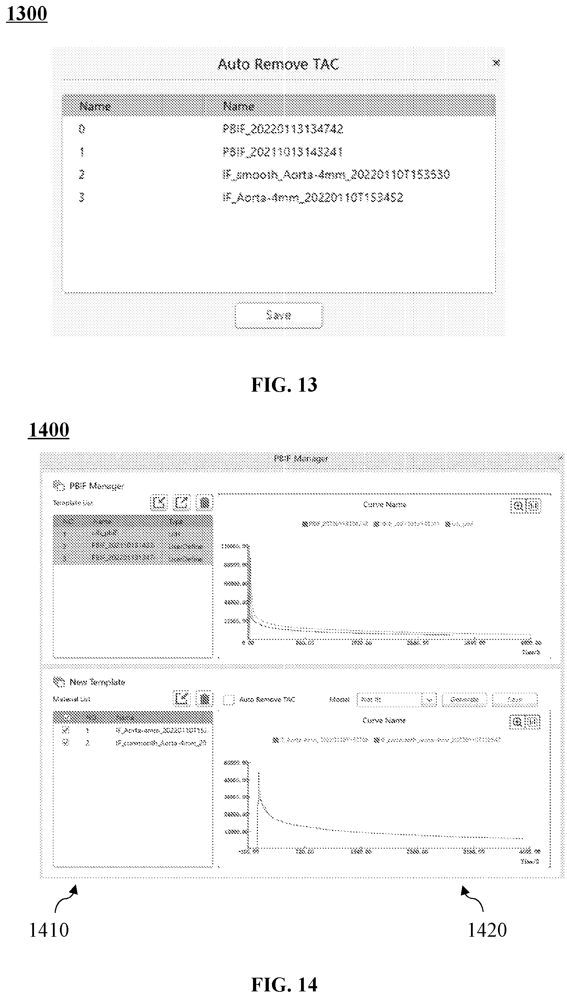

Refer to FIG. 13, FIG. 13 is a schematic diagram illustrating a third interface for determining an input function according to some embodiments of the present disclosure. In some embodiments, different time-activity curves may be characterized by names of the time-activity curves, and the eliminated time-activity curve(s) may be also displayed in a form of name. FIG. 13 shows the exemplary eliminated time-activity curves. It should be understood that the third interface may further display the target time-activity curve(s) that are not eliminated to reflect a screening result.

In some embodiments, if the time-activity curve(s) are not displayed by the form of name, the elimination result of the target time-activity curve(s) may be displayed in a corresponding manner, there is no specific limitation here.

In some embodiments, after the time-activity curves with obviously inconsistent shapes are eliminated, the elimination result may be displayed, so that the user can know which time-activity curves are eliminated. If there is a time-activity curve eliminated by mistake, the user can manually correct the mistake, and the elimination result can be more accurate.

In some embodiments, the displaying the second interface may further include the following operations: obtaining a second instruction and determine a smooth mode of the smooth operation based on the second instruction. In some embodiments, the second instruction may be used to instruct software to perform the smooth operation to the target input function curve based on a specific smooth mode.

It should be understood that the input function curve may include a variety of smooth modes, such as a non-smooth mode, an exponential smooth mode, a Feng model smooth mode, and other smooth modes. In some embodiments, the first interface may display options of a variety of smooth modes for a user to select, and the user may select one of the variety of smooth modes to generate the target input function curve.

In some embodiments, the variety of smooth modes may be displayed on the first interface by folding options, that is, in response to the second instruction of the user, a plurality of selection boxes of the smooth mode options may be popped up. Different smooth modes may be set in the plurality of selection boxes, and the user may select one of the selection boxes according to actual needs. It should be understood that the selection boxes popped up in response to the second instruction of the user may be in a form of a drop-down list, a thumbnail, etc., there is no specific limitation here.

In some embodiments, a variety of smooth modes of input function curves may be displayed, the user may select according to actual needs, and thus, actual needs can be accurately matched, and analysis results can be more accurate.

In some embodiments, the displaying the second interface may further include: displaying a second interface, the second interface presenting a target input function curve generated based on the one or more target time-activity curves and/or one or more historical input function curves.

It should be understood that the historical input function curve(s) may be preset by the user. In some embodiments, after the user selects the target time-activity curve(s), the historical input function curve(s) may be directly fitted with the target time-activity curve(s). The historical input function curve(s) may also be displayed in the first interface, and after the user selects the target time-activity curve(s) and/or the historical input function curve(s) at the same time, the historical input function curve(s) and the target time-activity curve(s) may be fitted, there is no specific limitation here.

FIG. 14 is a schematic diagram of a second interface for determining an input function according to some embodiments of the present disclosure. An operation interface of software may include one or more sub-interfaces at the same time. For example, as shown in FIG. 14, the operation interface in the figure may include an input function curve display interface, the first interface, and the second interface.

The lower left of the operation interface is the first interface, which may display names of the plurality of time-activity curves and historical input function curves. The user may select the target time-activity curve(s) or historical input function curve(s). An "automatically eliminate TAC" check box on the top of the first interface may be used to turn on a function of automatically eliminating one or more time-activity curves. If it is checked, the time-activity curve(s) with obvious inconsistent shapes may be eliminated. In addition, the operation interface may provide selections of the smooth modes of the target input function curve(s) in a form of folding options. By clicking a selection box next to "model selection", a plurality of selection boxes may pop up. Different curve fitting models may be set in the plurality of selection boxes. The user may select one of the selection boxes according to actual needs to determine a fitting model of the target input function curve(s). The curve fitting model may include a non-fitting model, a Feng model, and an exponential model, etc. It should be understood that when both the curve(s) and the smooth mode are selected, a generation of the target input function curve may be triggered by clicking a generate button, and the generated target input function curve may be displayed in the second interface at the lower right of the operation interface.

In some embodiments, the method for determining the input function may further include: displaying a fourth interface, the fourth interface presenting a comparison between at least one historical input function curve and one or more target input function curves. By comparing the historical input function curve(s) with a newly generated target input function curve on a same coordinate axis of the fourth interface, the change of the curve may be seen more intuitively.

In some embodiments, the historical input function curve(s) and the target time-activity curve(s) may be spliced to obtain a new input function curve. In some embodiments, the method for determining the input function may further include: obtaining a fourth instruction; in response to the fourth instruction, performing a splicing operation on the at least one historical input function curve and one of the one or more target time-activity curves; and/or displaying a fifth interface, the fifth interface presenting a result of the splicing operation.

In some embodiments, the fourth instruction may be used to instruct one historical input function curve in the target time-activity curve(s) to be automatically/manually spliced with another time-activity curve in the target time-activity curve(s) except the historical input function curve. In some embodiments, the fourth instruction may be related to a selection operation for selecting a first segment of the at least one historical input function curve and a second segment of the another time-activity curve.

In some embodiments, the fifth interface may further present a real-time process and/or a result of the selection operation, that is, the fifth interface may reflect a situation of the splicing of the two curves in real-time.

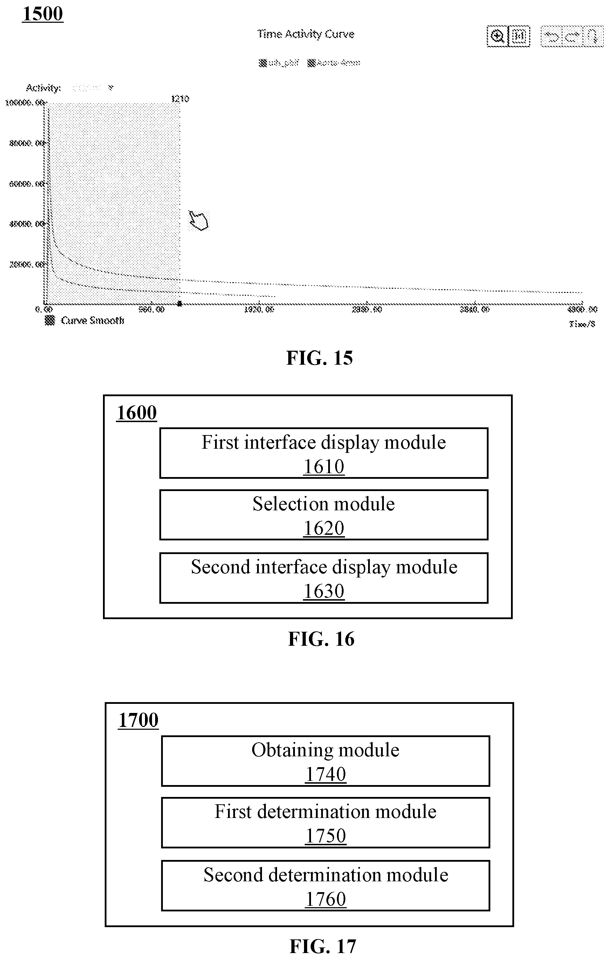

For example, refer to FIG. 15, the figure shows the fifth interface including a spliced input function curve in some embodiments. A selected range in the figure may be the input function curve selected by the user, and an unselected part (on the right side of the figure) is the time-activity curve selected by the user. When splicing, the user may drag a selected region back and forth to realize the splicing of two curves. Specifically, the user may select a dotted line box, use a cursor to pan and drag left and right, and after confirming a final position of the drag, the two curves may be spliced, and a new input function curve may be generated.

It should be understood that although operations in flowcharts according to the embodiments described above are sequentially displayed according to an arrow, the operations may be not necessarily sequentially executed according to an order indicated by the arrow. Unless explicitly stated in the present disclosure, there is no strict order limit on an execution of the operations, and the operations may be executed in other orders. Moreover, at least some of the operations in flowcharts according to the embodiments described above may include a plurality of operations or a plurality of stages, the plurality of operations or the plurality of stages are not necessarily executed at the same time, but may be executed at different times, and the execution order of the plurality of operations or the plurality of stages is not necessarily sequential, but may be performed alternately with other operations or at least part of operations or stages in other operations.

Based on the same inventive concept, embodiments of the present disclosure also provide a device for determining the input function to implement the method for determining the input function mentioned above. An implementation scheme for solving a problem provided by the device is similar to the implementation scheme recorded in the above method, so specific limitations in the embodiments of the one or more devices for determining the input function provided below can be seen in above definition of the method for determining the input function, and will not be repeated here.

In some embodiment, as shown in FIG. 16, a device for determining the input function may be provided. The device 1600 may include a first interface display module 1610, a selection module 1620, and a second interface display module 1630.

The first interface display module 1610 may be configured to display a first interface, wherein the first interface may include the plurality of time-activity curves.

The selection module 1620 may be configured to obtain the first instruction and select the target time-activity curve(s) based on the first instruction.

The second interface display module 1630 may be configured to display the second interface, wherein the second interface may include a target input function curve generated based on the target time-activity curve. The second interface display module 1630 may further be configured to display the second interface, wherein the second interface may include a target input function curve generated based on the target time-activity curve(s) and/or the historical input function curve(s).

The device 1600 may further include a screening module, an elimination module, and/or a smooth mode selection module.

The elimination module may be configured to screen based on peak time(s) of the time-activity curve(s), and determine the time-activity curve(s) whose peak time(s) are within a preset range as target time-activity curve(s). The elimination module may be further configured to determine an average peak time of the plurality of time-activity curves, and translate the plurality of time-activity curves based on the average peak time; and/or perform the screening operation based on the peak time(s) of the plurality of time-activity curves, and determine translated time-activity curve(s) whose peak time(s) are within a preset range as target time-activity curve(s). The elimination module may be further configured to display a third interface. The third interface may present a result of the screening operation.

The smooth mode selection module may be configured to obtain the second instruction, and determine the smooth mode of the target input function curve based on the second instruction.

In some embodiments, as shown in FIG. 17, a system 1700 for determining an input function may be provided. The system 1700 may include an obtaining module 1740, a third determination module 1750, and a fourth determination module 1760.

The obtaining module 1740 may be configured to obtain the plurality of time-activity curves. It should be understood that the plurality of time-activity curves may be the plurality of time-activity curves corresponding to the region of interest, and may specifically include the time-activity curves corresponding to the each voxel in the region of interest. It should be understood that the plurality of time-activity curves may be time-activity curves derived from historical data or time-activity curves generated in real-time, as long as the time-activity curves may characterize a relationship between concentration of a radioactive tracer and time in the region of interest, there is no specific limitation here.

The third determination module 1750 may be configured to determine target time-activity curve(s) based on the plurality of the time-activity curves. In the embodiment, the target time-activity curve(s) may be determined according to the user's instruction. In some embodiments, and the target time-activity curve(s) may be determined according to a preset condition.

The fourth determination module 1760 may be configured to determine the target input function curve based on the target time-activity curve(s).

It should be understood that an algorithm for generating the input function curve according to the time-activity curve fitting may be pre-stored in a server. Once the target time-activity curve(s) are determined, the corresponding target input function curve can be generated, and the input function can be obtained correspondingly for analysis.

Each module in the above device for determining the input function may be implemented in whole or in part by software, hardware and combinations thereof. The above modules may be embedded in or independent of a processor in the processing device in a form of hardware, or may be stored in a memory in the processing device in a form of software, so that the processor can call and execute the operations corresponding to the above modules.

The basic concepts have been described above. Obviously, for those skilled in the art, the above detailed disclosure is merely an example, and does not constitute a limitation of the present disclosure. Although there is no clear explanation here, those skilled in the art may make various modifications, improvements, and corrections for the present disclosure. This type of modification, improvement, and corrections are recommended in the present disclosure, so this class is corrected, improved, and the amendment remains in the spirit and scope of the exemplary embodiment of the present disclosure.

Meanwhile, the present disclosure uses specific words to describe embodiments of the present disclosure. As "one embodiment", "an embodiment", and/or "some embodiments" means a certain feature, structure, or characteristic of at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Further, certain features, structures, or features of one or more embodiments of the present disclosure may be combined.

Moreover, unless otherwise specified in the claims, the sequence of the present disclosure, the order of the sequence of the present disclosure, the use of digital letters, or other names are not used to define the order of the present disclosure processes and methods. Although some embodiments of the invention currently considered useful have been discussed through various examples in the above disclosure, it should be understood that such details are only for the purpose of illustration, and the additional claims are not limited to the disclosed embodiments. On the contrary, the claims are intended to cover all amendments and equivalent combinations in line with the essence and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the expression disclosed in the present disclosure and help the understanding of one or more invention embodiments, in the previous description of the embodiments of the present disclosure, a variety of features are sometimes combined into one embodiment, drawings or description thereof. However, the present disclosure method does not mean that the features needed in the spectrum ratio of this disclosure ratio are more characteristic. Rather, claimed subject matter may lie in less than all features of a single foregoing disclosed embodiment.

In some embodiments, the numbers expressing quantities of ingredients, properties, and so forth, used to describe and claim certain embodiments of the application are to be understood as being modified in some instances by the term "about," "approximate," or "substantially". Unless otherwise stated, "about," "approximate," or "substantially" may indicate ±20% variation of the value it describes. Accordingly, in some embodiments, the numerical parameters set forth in the description and attached claims are approximations that may vary depending upon the desired properties sought to be obtained by a particular embodiment. In some embodiments, the numerical parameters should be construed in light of the number of reported significant digits and by applying ordinary rounding techniques. Although the numerical domains and parameters used in the present disclosure are used to confirm its range breadth, in the specific embodiment, the settings of such values are as accurate as possible within the feasible range.

For each patent, patent application, patent application publication, or other materials cited in the present disclosure, such as articles, books, specifications, publications, documents, or the like, the entire contents of which are hereby incorporated into the present disclosure as a reference. Except for the application history documents that are inconsistent with or conflict with the contents of the present disclosure, and the documents that limit the widest range of claims in the present disclosure (currently or later attached to the present disclosure). It should be noted that if a description, definition, and/or terms in the subsequent material of the present disclosure are inconsistent or conflict with the content described in the present disclosure, the use of description, definition, and/or terms in this manual shall prevail.

Finally, it should be understood that the embodiments described in the present disclosure are intended to illustrate the principles of the embodiments of the present disclosure. Other embodiments may also be considered, as long as they do not deviate from the scope of this disclosure, as defined by the appended claims.

## Claims

1. A computer-implemented method for processing an image, comprising:
obtaining a plurality of dynamic frame images, each of the plurality of dynamic frame images at least including dynamic image information of a target region; and
determining a time-activity curve by inputting the plurality of dynamic frame images to a trained extraction model, wherein the trained extraction model is obtained by:
obtaining a plurality of training samples, each of the plurality of training samples including a sample dynamic image and a label of the sample dynamic image, the label including a sample time-activity curve corresponding to the sample dynamic image; and
training an initial extraction model, based on the plurality of training samples, to adjust model parameters of the initial extraction model to obtain the trained extraction model, **characterized in that**:
the sample dynamic image being obtained by segmenting a whole-body dynamic image based on an imaging range of the target region, the label being the sample time-activity curve corresponding to the whole-body dynamic image, and the label being obtained in a specific blood pool region, wherein the target region is a head region or an abdomen region, and the specific blood pool region is the aorta or the heart.

2. The method of claim 1, wherein the whole-body dynamic image is acquired by whole-body simultaneous imaging.

3. The method of claim 1 or 2, further comprising:
reconstructing a dynamic parametric image based on the plurality of dynamic frame images and the time-activity curve.

4. The method of any one of claims 1-3, further comprising:
displaying a first interface, the first interface presenting a plurality of time-activity curves, the plurality of time-activity curves including the time-activity curve;
obtaining a first instruction, and determining one or more target time-activity curves from the plurality of time-activity curves based on the first instruction; and
displaying a second interface, the second interface presenting a target input function curve generated based on the one or more target time-activity curves.

5. The method of claim 4, wherein the target input function curve is generated by:
performing a fitting operation on the one or more target time-activity curves to obtain the target input function curve.

6. The method of claim 5, further comprising:
obtaining another instruction; and
before the fitting operation, performing, based on the another instruction, a screening operation on the one or more target time-activity curves to remove an undesired time-activity curve that dissatisfies a preset rule.

7. The method of claim 6, wherein the preset rule includes that a starting time of each of the one or more target time-activity curves is within a first preset range and/or a starting time of each of the one or more target time-activity curves after translation according to an average peak time is within a second preset range.

8. The method of claim 6 or 7, wherein the one or more target time-activity curves include at least one historical input function curve.

9. The method of any one of claims 4-8, further comprising:
displaying a fourth interface, the fourth interface presenting a comparison between the at least one historical input function curve and the one or more target input function curves.

10. The method of any one of claims 4-9, wherein at least one time-activity curve among the plurality of time-activity curves is obtained by:
obtaining first positron emission tomography (PET) scan data acquired at a first time point and second PET scan data acquired at a second time point;
performing a reconstruction operation on the first PET scan data and the second PET scan data to generate a first initial PET image and a second initial PET image, respectively;
obtaining first information of a region of interest by segmenting the first initial PET image using a segmentation model;
obtaining second information of the region of interest by segmenting the second initial PET image using the segmentation model; and
generating the at least one time-activity curve based on the first information and the second information of the region of interest.

11. The method of any one of claims 4-10, wherein the plurality of time-activity curves include at least one time-activity curve uploaded to a preset system by a user.

12. A system for processing an image, comprising a dynamic image obtaining module (510) and a time-activity curve obtaining module (520), wherein:
the dynamic image obtaining module (510) is configured to obtain a plurality of dynamic frame images, each of the plurality of dynamic frame images at least includes dynamic image information of a target region; and
the time-activity curve obtaining module (520) is configured to determine a time-activity curve by inputting the plurality of dynamic frame images to a trained extraction model, wherein the trained extraction model is obtained by:
obtaining a plurality of training samples, each of the plurality of training samples including a sample dynamic image and a label of the sample dynamic image, the label including a sample time-activity curve corresponding to the sample dynamic image; and
training an initial extraction model, based on the plurality of training samples, to adjust model parameters of the initial extraction model to obtain the trained extraction model, **characterized in that**:
the sample dynamic image being obtained by segmenting a whole-body dynamic image based on an imaging range of the target region, the label being the sample time-activity curve corresponding to the whole-body dynamic image, and the label being obtained in a specific blood pool region, wherein the target region is a head region or an abdomen region, and the specific blood pool region is the aorta or the heart.

13. A non-transitory computer-readable medium, comprising at least one set of instructions, wherein when executed by at least one processor (210) of a computing device (200), the at least one set of instructions direct the at least one processor (210) to perform operations including:
obtaining a plurality of dynamic frame images, each of the plurality of dynamic frame images at least including dynamic image information of a target region; and
determining a time-activity curve by inputting the plurality of dynamic frame images to a trained extraction model, wherein the trained extraction model is obtained by:
obtaining a plurality of training samples, each of the plurality of training samples including a sample dynamic image and a label of the sample dynamic image, the label including a sample time-activity curve corresponding to the sample dynamic image; and
training an initial extraction model, based on the plurality of training samples, to adjust model parameters of the initial extraction model to obtain the trained extraction model, **characterized in that**:
the sample dynamic image being obtained by segmenting a whole-body dynamic image based on an imaging range of the target region, the label being the sample time-activity curve corresponding to the whole-body dynamic image, and the label being obtained in a specific blood pool region, wherein the target region is a head region or an abdomen region, and the specific blood pool region is the aorta or the heart.

## Patentansprüche

1. Computerimplementiertes Verfahren, zur Verarbeitung eines Bildes, umfassend:
Erhalten einer Vielzahl von dynamischen Einzelbildern, wobei jedes der Vielzahl von dynamischen Einzelbildern mindestens dynamische Bildinformationen eines Zielbereichs einschließt; und
Bestimmen einer Zeit-Aktivitäts-Kurve durch Eingabe der Vielzahl von dynamischen Einzelbildern in ein trainiertes Extraktionsmodell, wobei das trainierte Extraktionsmodell erhalten wird durch:
Erhalten einer Vielzahl von Trainingsbeispielen, wobei jedes der Trainingsbeispiele ein dynamisches Beispielbild und ein Label des dynamischen Beispielbildes einschließt, wobei das Label eine dem dynamischen Beispielbild entsprechende Zeit-Aktivitäts-Kurve einschließt; und
Trainieren eines anfänglichen Extraktionsmodells auf Basis der Vielzahl von Trainingsbeispielen, um die Modellparameter des anfänglichen Extraktionsmodells anzupassen, um das trainierte Extraktionsmodell zu erhalten, **dadurch gekennzeichnet, dass**:
das dynamische Beispielbild durch Segmentierung eines dynamischen Ganzkörperbildes auf Basis eines Bildgebungsbereichs des Zielbereichs erhalten wird, wobei das Label die dem dynamischen Ganzkörperbild entsprechende Zeit-Aktivitäts-Kurve ist und das Label in einem spezifischen Blutpoolbereich erhalten wird, wobei der Zielbereich ein Kopfbereich oder ein Bauchbereich ist und der spezifische Blutpoolbereich die Aorta oder das Herz ist.

2. Verfahren nach Anspruch 1, wobei das dynamische Ganzkörperbild durch simultane Ganzkörperbildgebung erfasst wird.

3. Verfahren nach Anspruch 1 oder 2, ferner umfassend:
Rekonstruieren eines dynamischen parametrischen Bildes auf Basis der Vielzahl dynamischer Einzelbilder und der Zeit-Aktivitäts-Kurve.

4. Verfahren nach einem der Ansprüche 1 bis 3, ferner umfassend:
Anzeigen einer ersten Schnittstelle, wobei die erste Schnittstelle eine Vielzahl von Zeit-Aktivitäts-Kurven darstellt, wobei die Vielzahl von Zeit-Aktivitäts-Kurven die Zeit-Aktivitäts-Kurve einschließt;
Erhalten einer ersten Anweisung und Bestimmen einer oder mehrerer Ziel-Zeit-Aktivitäts-Kurven aus der Vielzahl der Zeit-Aktivitäts-Kurven auf Grundlage der ersten Anweisung; und
Anzeigen einer zweiten Schnittstelle, wobei die zweite Schnittstelle eine Ziel-Eingabefunktionskurve darstellt, die auf der Grundlage einer oder mehrerer Ziel-Zeit-Aktivitäts-Kurven erzeugt wurde.

5. Verfahren nach Anspruch 4, wobei die Ziel-Eingangsfunktionskurve erzeugt wird, durch:
Ausführen einer Anpassungsoperation auf der einen oder mehreren Ziel-Zeit-Aktivitäts-Kurven, um die Ziel-Eingangsfunktionskurve zu erhalten.

6. Verfahren nach Anspruch 5, ferner umfassend:
Erhalten einer weiteren Anweisung; und
vor der Anpassungsoperation, Ausführen, basierend auf der weiteren Anweisung, einer Screening-Operation auf die eine oder mehreren Ziel-Zeit-Aktivitäts-Kurven, um eine unerwünschte Zeit-Aktivitäts-Kurve zu entfernen, die einer voreingestellten Regel nicht entspricht.

7. Verfahren nach Anspruch 6, wobei die voreingestellte Regel einschließt, dass der Startzeitpunkt jeder der einen oder mehreren Ziel-Zeit-Aktivitäts-Kurven innerhalb eines ersten voreingestellten Bereichs liegt, und/oder ein Startzeitpunkt jeder der einen oder mehreren Ziel-Zeit-Aktivitäts-Kurven nach der Verschiebung gemäß einem durchschnittlichen Spitzenzeitpunkt innerhalb eines zweiten voreingestellten Bereichs liegt.

8. Verfahren nach Anspruch 6 oder 7, wobei die eine oder mehrere Ziel-Zeit-Aktivitäts-Kurven mindestens eine historische Eingangsfunktionskurve einschließen.

9. Verfahren nach einem der Ansprüche 4 bis 8, ferner umfassend:
Anzeigen einer vierten Schnittstelle, wobei die vierte Schnittstelle einen Vergleich zwischen der mindestens einen historischen Eingangsfunktionskurve und der einen oder mehreren Ziel-Eingangsfunktionskurven darstellt.

10. Verfahren nach einem der Ansprüche 4 bis 9, wobei mindestens eine Zeit-Aktivitäts-Kurve aus der Vielzahl von Zeit-Aktivitäts-Kurven erhalten wird, durch:
Erhalten von ersten Positronen-Emissions-Tomographie-Scandaten (PET-Scandaten), die zu einem ersten Zeitpunkt erfasst werden, und zweiten PET-Scandaten, die zu einem zweiten Zeitpunkt erfasst werden;
Durchführen einer Rekonstruktionsoperation an den ersten PET-Scandaten und den zweiten PET-Scandaten, um jeweils ein erstes anfängliches PET-Bild und ein zweites anfängliches PET-Bild zu erzeugen;
Erhalten erster Informationen über einen Bereich von Interesse durch Segmentierung des ersten anfänglichen PET-Bildes mithilfe eines Segmentierungsmodells;
Erhalten zweiter Informationen über den Bereich von Interesse durch Segmentierung des zweiten anfänglichen PET-Bildes mithilfe des Segmentierungsmodells; und
Erzeugen der mindestens einen Zeit-Aktivitäts-Kurve auf Basis der ersten und zweiten Informationen über den Bereich von Interesse.

11. Verfahren nach einem der Ansprüche 4 bis 10, wobei die Vielzahl der Zeit-Aktivitäts-Kurven mindestens eine Zeit-Aktivitäts-Kurve einschließt, die von einem Benutzer in ein voreingestelltes System hochgeladen wurde.

12. System zur Verarbeitung eines Bildes, umfassend ein dynamisches Bilderfassungsmodul (510) und ein Zeit-Aktivitäts-Kurven-Erfassungsmodul (520), wobei:
das dynamische Bilderfassungsmodul (510) so konfiguriert ist, dass es eine Vielzahl dynamischer Einzelbilder erfasst, wobei jedes der Vielzahl von dynamischen Einzelbilder mindestens dynamische Bildinformationen eines Zielbereichs einschließt; und
das Zeit-Aktivitäts-Kurven-Erfassungsmodul (520) so konfiguriert ist, dass es eine Zeit-Aktivitäts-Kurve bestimmt, indem es die Vielzahl dynamischer Einzelbilder in ein trainiertes Extraktionsmodell eingibt, wobei das trainierte Extraktionsmodell erhalten wird, durch:
Erhalten einer Vielzahl von Trainingsbeispielen, wobei jedes der Trainingsbeispiele ein dynamisches Beispielbild und ein Label des dynamischen Beispielbildes einschließt, wobei das Label eine dem dynamischen Beispielbild entsprechende Zeit-Aktivitäts-Kurve einschließt; und
Trainieren eines anfänglichen Extraktionsmodells auf Basis der Vielzahl von Trainingsbeispielen, um die Modellparameter des anfänglichen Extraktionsmodells anzupassen, um das trainierte Extraktionsmodell zu erhalten, **dadurch gekennzeichnet, dass**:
das dynamische Beispielbild durch Segmentierung eines dynamischen Ganzkörperbildes auf Basis eines Bildgebungsbereichs des Zielbereichs erhalten wird, wobei das Label die dem dynamischen Ganzkörperbild entsprechende Zeit-Aktivitäts-Kurve darstellt und das Label in einem spezifischen Blutpoolbereich erhalten wird, wobei der Zielbereich ein Kopfbereich oder ein Bauchbereich ist und der spezifische Blutpoolbereich die Aorta oder das Herz ist.

13. Nichtflüchtiges, computerlesbares Medium, das mindestens einen Satz von Anweisungen umfasst, wobei der mindestens eine Satz von Anweisungen, wenn er von mindestens einem Prozessor (210) einer Computervorrichtung ausgeführt wird, den mindestens einen Prozessor (210) anweist, Operationen durchzuführen, die Folgendes einschließen:
Erhalten einer Vielzahl von dynamischen Einzelbildern, wobei jedes der Vielzahl von dynamischen Einzelbilder mindestens dynamische Bildinformationen eines Zielbereichs einschließt; und
Bestimmen einer Zeit-Aktivitäts-Kurve durch Eingabe der Vielzahl dynamischer Einzelbilder in ein trainiertes Extraktionsmodell, wobei das trainierte Extraktionsmodell wie folgt erhalten wird:
Erhalten einer Vielzahl von Trainingsbeispielen, wobei jedes der Trainingsbeispiele ein dynamisches Beispielbild und ein Label des dynamischen Beispielbildes einschließt, wobei das Label eine dem dynamischen Beispielbild entsprechende Zeit-Aktivitäts-Kurve einschließt; und
Trainieren eines anfänglichen Extraktionsmodells auf Basis der Vielzahl von Trainingsbeispielen, um die Modellparameter des anfänglichen Extraktionsmodells anzupassen, um das trainierte Extraktionsmodell zu erhalten, **dadurch gekennzeichnet, dass**:
das dynamische Beispielbild durch Segmentierung eines dynamischen Ganzkörperbildes auf Basis eines Bildgebungsbereichs des Zielbereichs gewonnen wird, wobei das Label die dem dynamischen Ganzkörperbild entsprechende Zeit-Aktivitäts-Kurve darstellt und das Label in einem spezifischen Blutpoolbereich erhalten wird, wobei der Zielbereich ein Kopfbereich oder ein Bauchbereich ist und der spezifische Blutpoolbereich die Aorta oder das Herz ist.

## Revendications

1. Procédé mis en œuvre par ordinateur pour le traitement d'une image, comprenant :
l'obtention d'une pluralité d'images de trames dynamiques, chacune de ces images de trames dynamiques incluant au moins des informations d'image dynamique d'une région cible ; et
la détermination d'une courbe temps-activité en introduisant une pluralité d'images de trames dynamiques dans un modèle d'extraction entraîné, dans lequel le modèle d'extraction entraîné est obtenu par :
l'obtention d'une pluralité d'échantillons d'entraînement, chacun de la pluralité d'échantillons d'entraînement incluant une image dynamique échantillon et une étiquette de l'image dynamique échantillon, l'étiquette incluant une courbe temps-activité correspondant à l'image dynamique échantillon ; et
l'entraînement d'un modèle d'extraction initial, basé sur la pluralité d'échantillons d'entraînement, afin d'ajuster des paramètres du modèle d'extraction initial pour obtenir le modèle d'extraction entraîné, **caractérisé en ce que** :
l'image dynamique de l'échantillon est obtenue en segmentant une image dynamique du corps entier en fonction d'une plage d'imagerie de la région cible, l'étiquette étant la courbe temps-activité de l'échantillon correspondant à l'image dynamique du corps entier, et l'étiquette étant obtenue dans une région spécifique du pool sanguin, dans lequel la région cible est une région de la tête ou une région de l'abdomen, et la région spécifique du pool sanguin est l'aorte ou le cœur.

2. Procédé selon la revendication 1, dans lequel l'image dynamique du corps entier est acquise par imagerie simultanée du corps entier.

3. Procédé selon la revendication 1 ou 2 comprenant en outre :
la reconstitution d'une image paramétrique dynamique à partir de la pluralité d'images de trames dynamiques et de la courbe temps-activité.

4. Procédé selon l'une quelconque des revendication 1-3, comprenant en outre :
l'affichage d'une première interface, la première interface présentant une pluralité de courbes temps-activité, la pluralité de courbes temps-activité incluant la courbe temps-activité ;
l'obtention d'une première instruction et la détermination d'une ou plusieurs courbes temps-activité cibles à partir de la pluralité de courbes temps-activité basées sur la première instruction ; et
l'affichage d'une deuxième interface, la deuxième interface présentant une courbe de fonction d'entrée cible générée à partir d'une ou plusieurs courbes temps-activité cibles.

5. Procédé selon la revendication 4, dans lequel la courbe de la fonction d'entrée cible est générée en :
effectuant une opération d'ajustement sur l'une ou plusieurs courbes temps-activité cibles afin d'obtenir la courbe de fonction d'entrée cible.

6. Procédé selon la revendication 5, comprenant en outre :
l'obtention d'une autre instruction ; et
avant l'opération d'ajustement, la réalisation, sur la base d'une autre instruction, d'une opération de filtrage sur une ou plusieurs courbes temps-activité cibles afin de supprimer une courbe temps-activité indésirable qui ne satisfait pas à une règle prédéfinie.

7. Procédé selon la revendication 6, dans lequel la règle prédéfinie inclut qu'une heure de début de chacune des courbes temps-activité cibles se situe dans une première plage prédéfinie et/ou l'heure de début de chacune des une ou plusieurs courbes temps-activité cibles, après traduction selon une heure de pointe moyenne, se situe dans une plage prédéfinie d'une seconde.

8. Procédé selon la revendication 6 ou 7, dans lequel la ou les courbes temps-activité cibles incluent au moins une courbe de fonction d'entrée historique.

9. Procédé selon l'une quelconque des revendications 4-8, comprenant en outre :
l'affichage d'une quatrième interface, la quatrième interface présentant une comparaison entre l'au moins une courbe de fonction d'entrée historique et les une ou plusieurs courbes de fonction d'entrée cibles.

10. Procédé selon l'une quelconque des revendications 4-9, dans lequel au moins une courbe temps-activité parmi la pluralité de courbes temps-activité est obtenue par :
l'obtention de premières données de tomographie par émission de positons (TEP) acquises à un premier moment et de deuxièmes données de tomographie par émission de positons (TEP) acquises à un deuxième moment ;
la réalisation d'une opération de reconstitution sur les données du premier PET scan et les données du deuxième PET scan afin de générer respectivement une première image PET initiale et une deuxième image PET initiale ;
l'obtention de premières informations sur une région d'intérêt en segmentant la première image TEP initiale à l'aide d'un modèle de segmentation ;
l'obtention de deuxièmes informations sur la région d'intérêt en segmentant la deuxième image TEP initiale à l'aide du modèle de segmentation ; et
la génération d'au moins une courbe temps-activité à partir des premières informations et des deuxièmes informations de la région d'intérêt.

11. Procédé selon l'une quelconque des revendications 4-10, dans lequel la pluralité de courbes temps-activité inclut au moins une courbe temps-activité téléchargée sur un système prédéfini par un utilisateur.

12. Système de traitement d'image, comprenant un module d'acquisition d'image dynamique (510) et un module d'acquisition de courbe temps-activité (520), dans lequel :
le module d'acquisition d'images dynamiques (510) est configuré pour obtenir une pluralité d'images de trames dynamiques, chacune de ces images de trames dynamiques incluant au moins des informations d'image dynamique d'une région cible ; et
le module d'obtention de la courbe temps-activité (520) est configuré pour déterminer une courbe temps-activité en fournissant la pluralité d'images de trames dynamiques à un modèle d'extraction entraîné, dans lequel le modèle d'extraction entraîné est obtenu par :
l'obtention d'une pluralité d'échantillons d'entraînement, chacun de la pluralité d'échantillons d'entraînement incluant une image dynamique échantillon et une étiquette de l'image dynamique échantillon, l'étiquette incluant une courbe temps-activité correspondant à l'image dynamique échantillon ; et
l'entraînement d'un modèle d'extraction initial, basé sur la pluralité d'échantillons d'entraînement, afin d'ajuster des paramètres du modèle d'extraction initial pour obtenir le modèle d'extraction entraîné, **caractérisé en ce que** :
l'image dynamique de l'échantillon est obtenue en segmentant une image dynamique du corps entier en fonction d'une plage d'imagerie de la région cible, l'étiquette étant la courbe temps-activité de l'échantillon correspondant à l'image dynamique du corps entier, et l'étiquette étant obtenue dans une région spécifique du pool sanguin, dans lequel la région cible est une région de la tête ou une région de l'abdomen, et la région spécifique du pool sanguin est l'aorte ou le cœur.

13. Support lisible sur ordinateur non transitoire, comprenant au moins un ensemble d'instructions, dans lequel lorsqu'il est exécuté par au moins un processeur (210) d'un dispositif informatique (200), le au moins un ensemble d'instructions ordonne à l'au moins un processeur (210) d'effectuer des opérations incluant :
l'obtention d'une pluralité d'images de trames dynamiques, chacune de ces images de trames dynamiques incluant au moins des informations d'image dynamique d'une région cible ; et
la détermination d'une courbe temps-activité en introduisant une pluralité d'images de trames dynamiques dans un modèle d'extraction entraîné, dans lequel le modèle d'extraction entraîné est obtenu par :
l'obtention d'une pluralité d'échantillons d'entraînement, chacun de la pluralité d'échantillons d'entraînement incluant une image dynamique échantillon et une étiquette de l'image dynamique échantillon, l'étiquette incluant une courbe temps-activité correspondant à l'image dynamique échantillon ; et
l'entraînement d'un modèle d'extraction initial, basé sur la pluralité d'échantillons d'entraînement, afin d'ajuster des paramètres du modèle d'extraction initial pour obtenir le modèle d'extraction entraîné, **caractérisé en ce que** :
l'image dynamique de l'échantillon est obtenue en segmentant une image dynamique du corps entier en fonction d'une plage d'imagerie de la région cible, l'étiquette étant la courbe temps-activité de l'échantillon correspondant à l'image dynamique du corps entier, et l'étiquette étant obtenue dans une région spécifique du pool sanguin, dans lequel la région cible est une région de la tête ou une région de l'abdomen, et la région spécifique du pool sanguin est l'aorte ou le cœur.
